(19) Europäisches Patentamt
European Patent Office
Office européen des brevets

(11) Veröffentlichungsnummer: **0 011 047**
**B1**

(12) # EUROPÄISCHE PATENTSCHRIFT

(45) Veröffentlichungstag der Patentschrift: **08.02.84**

(21) Anmeldenummer: **79810077.2**

(22) Anmeldetag: **27.08.79**

(51) Int. Cl.³: **A 01 N 25/32,** A 01 N 37/50, A 01 N 43/28, A 01 N 43/32, C 07 C 131/00, C 07 D 211/16, C 07 D 317/22, C 07 D 319/06

(54) **Oxim-Derivate zum Schutz von Pflanzenkulturen.**

(30) Priorität: **01.09.78 CH 9252/78**

(43) Veröffentlichungstag der Anmeldung:
**14.05.80 Patentblatt 80/10**

(45) Bekanntmachung des Hinweises auf die Patenterteilung:
**08.02.84 Patentblatt 84/6**

(84) Benannte Vertragsstaaten:
**AT BE CH DE FR GB IT NL**

(56) Entgegenhaltungen:
**DE - A - 2 808 317**
**DE - B - 1 567 075**
**FR - A - 2 322 861**

(73) Patentinhaber: **CIBA-GEIGY AG**
**Patentabteilung Postfach**
**CH-4002 Basel (CH)**

(72) Erfinder: **Szczepanski, Henry, Dr.**
**Waldshuterstrasse 55**
**CH-4310 Rheinfelden (CH)**
Erfinder: **Martin, Henry, Dr.**
**Jupiterstrasse 17**
**CH-4123 Allschwil (CH)**
Erfinder: **Föry, Werner, Dr.**
**Benkenstrasse 65**
**CH-4054 Basel (CH)**
Erfinder: **Pissiotas, Georg, Dr.**
**Breslauerstrasse 8**
**D-7850 Lörrach (DE)**

**EP 0 011 047 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Oxim-Derivate zum Schutz von Pflanzenkulturen

Die vorliegende Erfindung betrifft ein Verfahren zum Schutz von Pflanzenkulturen vor der phytotoxischen Wirkung starker Herbizide mittels Oxim-Derivaten sowie einige neue Oxim-Derivate.

Die als Wirkstoff zu verwendenden Oxim-Derivate entsprechen der Formel I

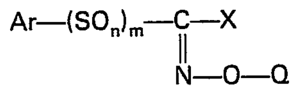

(I)

worin

n 0, 1 oder 2 und m 0 oder 1 ist, und worin

Ar einen Phenylrest

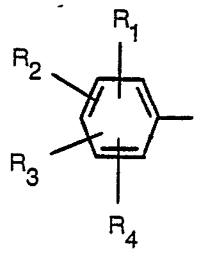

einen durch $R_2$ und $R_3$ substituierten Naphthylrest,

einen 5- bis 10-gliedrigen heterocyclischen Rest, der maximal 3 gleiche oder verschiedene Heteroatome N, O und/oder S enthält und durch $R_2$, $R_3$ und $R_4$ substituiert ist und durch Oxo der Thiono substituiert sein kann, bedeutet, oder worin, wenn m = 0 ist,

Ar einen Rest R—CO— darstellt, worin

R einen Rest $—OR_5$ bedeutet, worin $R_5$ für eine aliphatische Gruppe mit maximal 8 C-Atomen oder araliphatische Gruppe mit maximal 15 C-Atomen oder eine cycloaliphatische oder aromatische Gruppe mit je maximal 10 C-Atomen steht, wobei als Substituenten der aromatischen Reste oder des cycloaliphatischen Restes Halogen, —CN, $—NO_2$, Niederalkyl, Niederalkoxy, Halogenalkyl in Frage kommen,

ein Rest $—NH—CO—NH—R_7$ oder

ein Rest $—N(R_6)$ $(R_7)$ ist, worin $R_6$ für Wasserstoff oder Niederalkyl steht und $R_7$ Wasserstoff oder eine aliphatische Gruppe mit maximal 8 C-Atomen oder eine araliphatische Gruppe mit maximal 15 C-Atomen oder eine cycloaliphatische oder aromatische Gruppe mit je maximal 10 C-Atomen bedeutet, wobei als mögliche Substituenten der aromatischen Gruppe oder des cycloaliphatischen Restes Halogen, —CN, $NO_2$, Niederalkyl, Niederalkoxy, Halogenalkyl in Frage kommen,

einen Rest $—N(R_6)(R_7)$, wobei $R_6$ und $R_7$ zusammen mit dem N-Atom einen 5- oder 6-gliedrigen heterocyclischen Ring bilden, der als mögliches weiteres Heteroatom noch Sauerstoff enthalten kann,

$R_1$ Wasserstoff, Halogen, Niederalkyl, Niederalkoxy oder einen gegebenenfalls maximal zweimal durch Halogen, CN, $NO_2$, $CF_3$ substituierten paraständigen Phenoxyrest darstellt,

$R_2$, $R_3$, $R_4$ unabhängig voneinander Wasserstoff, Halogen, CN, $NO_2$, Niederalkyl, Niederalkoxy, Halogenalkyl, Halogenalkoxy, Niederalkanoyl, OH, Phenyl, Halogenphenyl, Niedercarbalkoxy, Niederalkoxycarbonyl, Niederalkoxycarbonyloxy, Niedercarbamoyloxy, Niederalkylthio, Niederalkylsulfonyl, Phenalkoxy, Cyclohexyl, $NH_2$, —NH-Niederalkyl, $—N(Niederalkyl)_2$, Niederalkanoylamino, Carbonsäureamid, Sulfonsäureamid bedeutet,

X für Wasserstoff, —CN, Halogen, Niederalkyl, Niederalkanoyl, —COOH, einen Carbonsäureesterrest, einen Carbonsäureamidrest steht, und

Q für den Rest $—C_aH_{2a}—R_8$ steht,

worin

a für eine ganze Zahl zwischen 1 und 6 steht, wobei der entsprechende Rest auch verzweigt sein kann, und $R_8$ für eine der nachfolgenden Gruppen steht.

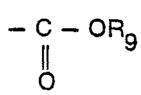 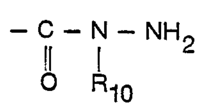 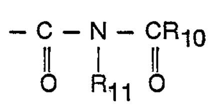 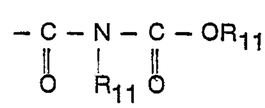

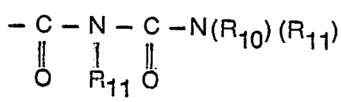 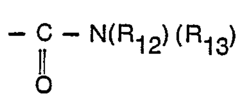 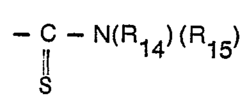 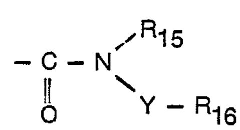

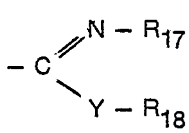 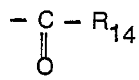 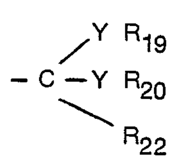

wobei die Substituenten $R_9$ bis $R_{20}$, $R_{22}$ und Y folgende Definitionen haben:

$R_9$ Wasserstoff oder Kation einer Base $^1/_p$ $M^{p\oplus}$ wobei

M eine Alkali- Erdalkali-Kation oder ein Fe-, Cu-, Zn-, Mn-, Co-, Ni-Kation oder einen Amino-Rest

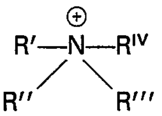

bedeuten, und

p als ganze Zahl 1, 2 oder 3 die Wertigkeit des Kations berücksichtigt, während R', R'', R''' und $R^{IV}$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten,

$R_{10}$ Wasserstoff, ein aliphatischer Rest, ein araliphatischer Rest oder ein gegebenenfalls ein- oder mehrfach durch Halogen, Halogenalkyl, Niederalkoxy und/oder Cyano substituierter aromatischer Rest

$R_{11}$ Wasserstoff, Niederalkyl

$R_{12}$ und $R_{13}$ zusammen mit dem Stickstoffatom ein gegebenenfalls ein- oder mehrfach durch Halogen, Cyano und/oder Niederalkyl substituierter und gegebenenfalls durch ein N, O oder S unterbrochener 5- bis 6-gliedriger Ring,

$R_{14}$ ein cycloaliphatischer oder ein araliphatischer oder aliphatischer Rest oder ein gegebenenfalls ein- oder mehrfach durch Halogen, Halogenalkyl, Niederalkoxy und/oder Cyano substituierter aromatischer Rest,

$R_{15}$ Wasserstoff, Niederalkyl oder Cycloalkyl,

$R_{16}$ ein gegebenenfalls durch Cyano substituierter aliphatischer Rest oder ein gegebenenfalls ein- oder mehrfach durch Halogen, Halogenalkyl, Niederalkoxy und/oder Cyano substituierter aromatischer Rest

$R_{17}$ Wasserstoff gegebenenfalls durch Halogen substituiertes Niederalkenyl oder Niederalkinyl, Niederalkyl oder Cycloalkyl,

$R_{18}$ Niederalkyl oder Cycloalkyl,

$R_{17}$ und $R_{18}$ zusammen mit —N=C—Y— ein gegebenenfalls durch Niederalkyl substituierter, 5- bis 6-gliedriger Ring,

$R_{19}$ und $R_{20}$ unabhängig voneinander Niederalkyl oder zusammen mit —Y—C—Y— ein gegebenenfalls durch Niederalkyl, Halogen und/oder Nitro substituierter 5- bis 6-gliedriger Ring,

$R_{22}$ Wasserstoff, ein cycloaliphatischer oder ein araliphatischer oder aliphatischer Rest oder ein gegebenenfalls ein- oder mehrfach durch Halogen, Halogenalkyl, Niederalkoxy und/oder Cyano substituierter aromatischer Rest,

Y Sauerstoff oder Schwefel ist.

In der Formel I ist unter Halogen Fluor, Chlor, Brom oder Jod zu verstehen.

Carbonsäureester sind Carbonsäureniederalkylester. Carbonsäureamide bedeuten neben —$CONH_2$ auch monoalkylsubstituierte oder symmetrisch oder unsymmetrisch dialkylsubstituierte Amid wobei die Alkylgruppen Niederalkyl darstellen.

Der Ausdruck Alkyl allein oder als Teil eines anderen Substituenten umfasst verzweigte oder unverzweigte $C_1$- bis $C_8$-Alkylgruppen; Niederalkyl allein oder als Teil eines anderen Substituenten bedeutet $C_1$—$C_4$ Alkyl. Beispiele sind Methyl, Aethyl, Propyl, Isopropyl, Butyl, Isobutyl, sec. Butyl, tert. Butyl, sowie die höheren Homologen Amyl, Isoamyl, Hexyl, Heptyl, Octyl samt ihren Isomeren. Sinngemäss enthalten Alkanoyle oder Cyanalkyle ein zusätzliches C-Atom. Entsprechend enthalten niedere Alkenyl- oder Alkinylgruppen maximal 4 C-Atome.

Der Begriff aliphatische Gruppe schliesst gesättigte (Alkyle) wie auch ungesättigte (Alkenyle, Alkadienyle, Alkinyle), halogensubstituierte, cyanosubstituierte und durch Sauerstoff unterbrochene Reste ein, die maximal 8 Kohlenstoffatome enthalten.

Der Begriff aromatische Gruppe umfasst Phenyl und Naphthyl, die grundsätzlich ein- oder mehrfach durch CN, $NO_2$, Halogen, Niederalkyl, Niederalkoxy oder Halogenalkyl substituiert sein können. Ein araliphatischer Rest umfasst ein gegebenenfalls ein- bis dreifach substituiertes Phenyl oder Naphtyl, das über Niederalkyl oder Niederalkenyl an den Rest des Moleküls gebunden ist. Beispiele sind die Grundkörper Benzyl, Phenäthyl, Phenylallyl sowie Homologe.

Gegebenenfalls substituierte heterocyclische Reste können mono- oder bicyclisch sein. Als Beispiel seien genannt: Furan, Nitrofuran, Bromfuran, Methylfuran, Thiophen, Chlorthiophen, Pyridin, 2,6-Dichlorpyridin, Pyrimidin, Pyridazin, Pyrazin, Piperidin, Methylpiperidin, Morpholin, Thiomorpholin, Tetrahydrofuran, Oxazol, Pyrazol, Pyrrol, Pyrrolin, Pyrrolidin, Thiazol, 2,3-Dihydro-4-H-pyran, Pyran, Dioxan, 1,4-Oxathi-(2)-in, Benzthiazol, Benzoxazol, Benzimidazol, Chinolin, Benz-1,3-dioxolan, Cycloalkylgruppen sind Cyclopropyl, Cyclobutyl, Cylopentyl, Cyclohexyl und Cycloheptyl. Cycloaliphatische Reste entsprechen diesen Ringsystemen, können daneben aber noch, je nach Möglichkeit, eine oder mehrere Doppelbindungen enthalten.

Die Verbindungen der Formel I können hergestellt werden, indem man

a) mit Ausnahme der Verbindungen, worin $R_8$ für 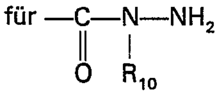

steht eine Verbindung der Formel II 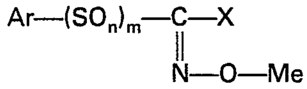

mit einer Verbindung der Formel III $Hal'—Q$ (III)

umsetzt oder

b) in den Fällen, wo $R_8$ für 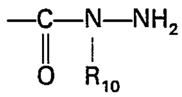 oder 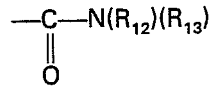

eine Verbindung der Formel IV 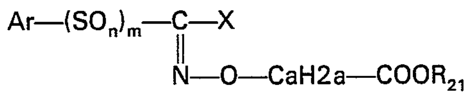 (IV)

mit einer Verbindung der Formel V oder VI

(V) 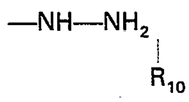 $—HN(R_{12})(R_{13})$ (VI)

umsetzt, wobei in den Formeln II, III, IV, V und VI Ar, X, Q, m und n die für Formel I gegebenen Bedeutungen haben, Hal' für Halogen, vorzugsweise Chlor oder Brom, Me für Wasserstoff oder ein Metallkation, vorzugsweise ein Alkali- oder Erdalkalikation, und $R_{21}$ für Wasserstoff oder Niederalkyl stehen.

Die Verbindungen der Formel IV können analog zum Verfahren

a) hergestellt werden.

Die Umsetzungen können in An- oder Abwesenheit von gegenüber den Reaktionsteilnehmern inerten Lösungsmitteln durchgeführt werden. Es kommen beispielsweise folgende in Frage: Alkohole wie Aethanol: Ketone wie Aceton: Nitrile wie Acetonitril: N,N-dialkylierte Amide wie Dimethylformamid: Dimethylsulfoxid, Pyridine sowie Gemische solcher Lösungsmittel untereinander.

In den Fällen wo Me für Wasserstoff steht, wird das Verfahren in Gegenwart einer Base durchgeführt. Beispiele geeigneter Basen sind anorganische Basen wie die Oxide, Hydroxy, Hydride, Carbonate und Hydrogencarbonate von Alkali und Erdalkalimetallen, sowie z.B. tertiäre Amine wie Trialkylamine (z.B. Triäthylamin), Pyridin usw.

Die Reaktionstemperaturen liegen zwischen 0—150°C, vorzugsweise 10—80°C. Die Reaktionen werden bei Normaldruck und bei a) gegebenenfalls unter einer Stickstoffatmosphäre durchgeführt.

Die Verbindungen der Formel II werden analog an sich bekannter Methoden hergestellt. Die Verfahren a) und b) bilden auch einen Teil der Erfindung.

Verbindungen der Formel I können grundsätzlich auch nach anderen an sich bekannten Methoden hergestellt werden (vgl. J. für Prakt. Chemie *66* p. 353; Pharm. Zentr. Halle *55*, p 735; J. Med. Chem. *20* p 1199).

Salze werden ebenfalls nach an sich bekannten Methoden hergestellt.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%) Granulate, Umhüllungsgranulate, Imprägnierungsgranulate und Homogengranulate, Pellets (Körner) 1 bis 80%);

Flüssige Aufarbeitungsformen:

a) in Wasser dispergierbare Wirkstoffkonzentrate:

Spritzpulver (wettable powders) and Pasten (25—90% in der Handelspackung, 0,01 bis 15% in gebrauchsfertiger Lösung); Emulsions- und Lösungskonzentrate (10 bis 50%; 0,01 bis 15% in gebrauchsfertiger Lösung);

b) Lösungen (0,1 bis 20%); Aerosole

Solche Mittel gehören ebenfalls zur Erfindung.

Als Gegenmittel oder Antidote sind schon verschiedene Stoffe vorgeschlagen worden, welche befähigt sind, die schädigenden Wirkungen eines Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei kann ein solches Gegenmittel, auch Safener genannt, je nach seinen Eigenschaften zur Vorbehandlung des Saatgutes der Kulturpflanze (Beizung des Samens oder der Stecklinge) oder vor der Saat in die Saatfurchen oder als Tankmischung für sich allein oder zusammen mit dem Herbizid vor oder nach dem Auflaufen der Pflanzen verwendet werden. Vorauflauf-Behandlung schliesst sowohl die Behandlung der Anbaufläche vor der Aussaat (ppi = "pre plant incorporation") als auch die Behandlung der angesäten, aber noch nicht bewachsenen Anbauflächen ein.

So beschreibt die GB—PS 1 277 557 die Behandlung von Samen bzw. Sprösslingen von Weizen und Sorghum mit gewissen Oxamsäureestern und Amiden vor dem Angriff durch N-Methoxy-methyl-2',6'-diäthyl-chloracetanilid (Alachlor). Andere Literaturstellen (DE—OS 1 952 910, DE—OS 2 245 471, FR—PS 2 021 611) schlagen Gegenmittel zur Behandlung von Getreide, Mais- und Reis-Samen zum Schutz gegen den Angriff herbizider Thiolcarbamate vor. In der DE—PS 1 567 075 und der US—PS 3 131 509 werden Hydroxyamino-acetanilide und Hydantoine für den Schutz von Getreidesamen gegenüber Carbamaten wie IPC, CIPC etc. vorgeschlagen. In der weiteren Entwicklung haben sich alle diese Präparate jedoch als ungenügend erwiesen.

Ueberraschenderweise besitzen Oxime der Formel I die Eigenschaft, Kulturpflanzen vor dem Angriff pflanzenaggressiver Agrarchemikalien zu schützen, insbesondere vor Herbiziden der verschiedensten Stoffklassen, darunter 1,3,5-Triazinen, 1,2,4-Triazinonen, Phenylharnstoffderivaten, Carbamaten, Thiolcarbamaten, Phenoxyessigsäureestern, Phenoxypropionsäureestern, Halogenacetaniliden, Halogenphenoxyessigsäureestern, subst. Phenoxyphenoxyessigsäureestern und -propionsäureestern, subst. Pyridinoxyphenoxy-essigsäureestern und -propionsäureestern, Benzoesäurederivaten usw., sofern diese nicht oder ungenügend kulturentolerant sind.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid vor oder nach dem Auflaufen der Pflanzen appliziert werden. Die Behandlung der Pflanze oder des Saatguts mit dem Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = "pre plant incorporation") als auch die Bahandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, verhalten sich die Mengen von Antidote der Formel I zu phytotoxischer Chemikalie wie 1:100 bis 5:1, bevorzugt 1:20 bis 1:1. Bei Samenbeizung und ähnlichen gezielten Schutzmassnahmen werden jedoch weit geringere Mengen Antidote im Vergleich mit den z.B. später pro Hektar Anbaufläche verwendeten Herbizidmengen benötigt (z.B. ca. 1:3000 bis 1:1000). In der Regel stehen protektive Massnahmen wie Samenbeizung mit einem Antidote der Formel I und mögliche spätere Feldbehandlung mit Agrarchemikalien nur in losem Zusammenhand. Vorbehandeltes Saat- und Pflanzengut kann später in Landwirtschaft, Garenbau und Forstwirtschaft mit unterschiedlichen Chemikalien in Berührung kommen.

Die Erfindung bezieht sich daher auch auf kulturpflanzen-protektive Mittel, die als Wirkstoff lediglich ein Antidote der Formel I zusammen mit üblichen Trägerstoffen enthalten. Solche Mittel können gegebenenfalls zusätzlich mit jenem Herbizid gemischt sein, vor dessen Einfluss die Kulturpflanze geschützt werden soll.

Kulturpflanzen seien im Rahmen vorliegender Erfindung alle Pflanzen, die in irgendeiner Form Ertragsstoffe produzieren (Samen, Wurzeln, Stengel, Knollen, Blätter, Blüten, Inhaltsstoffe wie Oele, Zucker, Stärke, Eiweiss etc.) und zu diesem Zweck angebaut und gehegt werden. Zu diesen Pflanzen gehören beispielsweise sämtliche Getreidearten, Mais, Reis, Edelhirse, Soja, Bohnen, Erbsen, Kartoffeln, Gemüse, Baumwolle, Zuckerrüben, Zuckerrohr, Erdnüsse, Tabak, Hopfen, dann jedoch auch Zierpflanzen, Obstbäume sowie Bananen, Kakao- und Naturkautschuk-Gewächse. Diese Aufzählung stellt keine Limitierung dar. Grundsätzlich lässt sich ein Antidote überall dort einsetzen, wo eine Kulturpflanze vor der Phytotoxizität einer Chemikalie geschützt werden soll.

Die Erfindung bezieht sich auch auf ein Verfahren zum Schutz von Kulturpflanzen vor aggressiven (phytotoxischen) Herbiziden, indem ein als Antidote wirkendes Oximderivat der Formel I wahlweise vor oder nach Applikation des Herbizids oder auch gleichzeitig mit dem Herbizid angewendet wird.

Die Erfindung bezieht sich ferner auf das Vermehrungsgut solcher Kulturpflanzen, das protektiv mit einem Oximderivat der Formel I behandelt wurde. Unter dem Begriff "Vermehrungsgut" sind alle generativen Pflanzenteile zu verstehen, die zur Vermehrung der Kulturpflanze eingesetzt werden können. Dazu zählen Samenkörner (Saatgut im engeren Sinne), Wurzeln, Früchte, Knollen, Rhizome, Stengelteile, Zweige (Stecklinge) und andere Pflanzenteile. Es zählen aber auch angekeimte Pflanzen und Jungpflanzen dazu, die nach der Ankeimung oder dem Auflaufen weiterverpflanzt werden sollen.

Solche Jungpflanzen lassen sich durch eine vollständige oder partielle Tauchbehandlung vor dem Weiterverpflanzen gezielt schützen.

Die nachfolgenden Substituententypen oder Kombinationen dieser untereinander werden bevorzugt:

Bei Q:—

a) $CH_2CONHNH_2$

b) 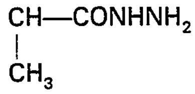

c) $C_aH_{2a}CONHNH_2$

d) 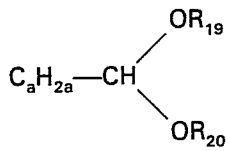

e) 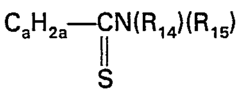

f) 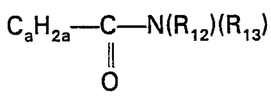

Bei a: 1 oder 2

Bei Ar:—

a) 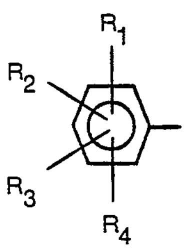

b) 1-Naphthyl

c) 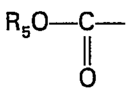

d) Benzoxazol

e) Benzthiazol

f) 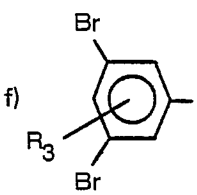

Bei X:—

a) Cyano

b) Wasserstoff

c) ein Carbonsäureesterrest

d) Niederalkyl

Bei n und m:—

a) n = 2 m = 1

b) m = 0

Eine Gruppe von Verbindungen, welche der allgemeinen Formel

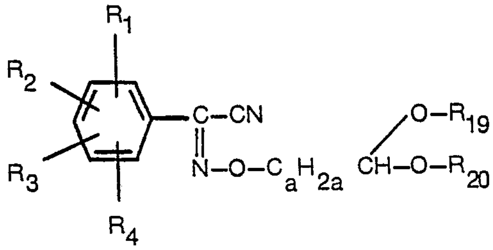

entsprechen, worin a, $R_1$, $R_2$, $R_3$, $R_4$, $R_{19}$ und $R_{20}$ die unter Formel I gegebene Bedeutung haben, besitzen überdies eine das Pflanzenwachstum hemmende Wirkung. Sie eignen sich besonders zur Hemmung

des vegetativen Wachstums von Sojapflanzen, ohne dabei auch das generative Wachstum zu beeinträchtigen. Dies führt zu kleineren, gedrungenen Pflanzen, die den Klima- und Witterungsinflüssen weniger ausgesetzt sind und pro Anbaufläche mehr Ertrag abwerfen, einerseits weil weniger Energie für das vegetative Wachstum verwendet werden muss, anderseits weil es möglich ist kleinwüchsige Pflanzen näher zusammen anzubauen.

Die nachfolgenden Beispiele dienen zur näheren Erläuterung der Erfindung ohne dieselbe einzuschränken. Die Temperaturen sind in Celsiusgraden, die Druckangaben in Millibar angegeben. Prozente und Teile beziehen sich auf Gewicht.

## Beispiel 1

Herstellung von

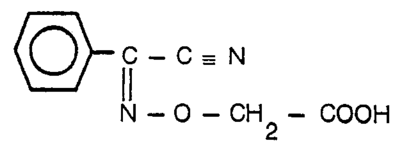

33,6 g des Natriumsalzes von $\alpha$-Phenylacetonitriloxim wurden in 200 ml Acetonitril suspendiert, mit 23 g Chloressigsäure Natrium-Salz in 20 ml Wasser versetzt und 1 Std. am Rückfluss gerührt. Danach wurde heiss filtriert und das Filtrat eingedampft, worauf ein halbfester Rückstand blieb. Dieser Rückstand wurde mit ca. 50 ml kaltem Wasser verrührt, abgenutscht und getrocknet.

Es wurden 30,3 g von

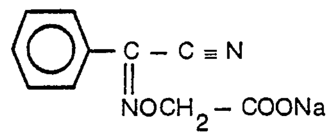

erhalten.

Dieses Produkt wurde in Wasser heiss gelöst, mit Kohle verrührt und klarfiltriert. Das Filtrat wurde mit 2n HCl angesäuert, worauf ein weisser Niederschlag ausfiel, der abgenutscht, gewaschen und getrocknet wurde. Smp. 169—171°.

## Beispiel 2

Herstellung von

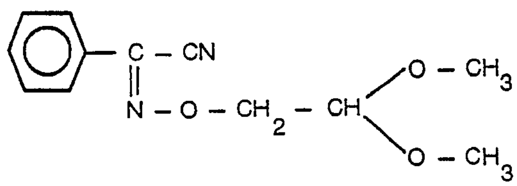

Zu einer Lösung von 16,8 g (0.1 Mol) des Natriumsalzes von $\alpha$-Phenylacetonitriloxim in 70 ml Dimethylsulfoxid wurden 16,9 g (0.1 Mol) Bromacetaldehyddimethylacetal gegeben und das Reaktionsgemisch 4 Std. auf 60° gewärmt. Anschliessend wurde mit 400 ml Aether versetzt und dreimal mit 2-proz. NaOH (je 200 ml) gewaschen.

Die Aetherlösung wurde eingedampft und das Rohprodukt aus Hexan umkristallisiert.

Erhalten wurden 14 g Kristalle Smp. 35°.

## Beispiel 3

Herstellung von

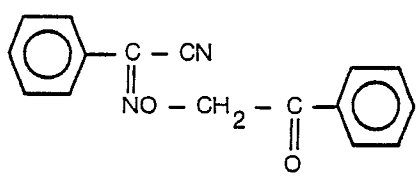

10 g $\omega$-Brom-acetophenon wurden in 70 ml Acetonitril gelöst und mit 8,5 g des Natriumsalzes von $\alpha$-Phenylacetonitriloxim versetzt. Die Temperatur wurde durch Kühlen zwischen 30° und 35° gehalten, und anschliessend wurde 2 Std. bei Raumtemperatur ausgerührt. Das Reaktionsgemisch wurde zur Trockne eingedampft, mit 200 ml Aether aufgenommen, dann zweimal mit je 200 ml 3-proz. NaOH-Lösung gewaschen, anschliessend mit $MgSO_4$ getrocknet und eingedampft.

Das Rohprodukt wurde aus Aether/Hexan umkristallisiert. Es wurden 6 g hellgelbe Kristalle vom Smp. 80° erhalten.

Beispiel 4

Herstellung von

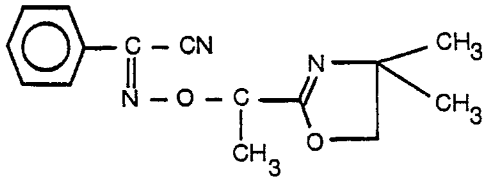

Zu einer Lösung von 15,4 g (0.1 Mol) des Natriumsalzes von $\alpha$-Phenylacetonitriloxim in 70 ml Dimethylsulfoxid wurden bei 20° 20,6 g (0,1 Mol) 2-[1-Bromäthyl]-4,4-dimethyloxazolin zugetropft. Nach Abklingen der schwach exothermen Reaktion wurde 24 Std. ausgerührt. Anschliessend wurde mit 100 ml Aether verdünnt und das Dimethylsulfoxid mit 3 x 300 ml Wasser ausgewaschen. Die Aetherphase wurde getrocknet und eingedampft. Das entstandene Kristallisat wurde mit Hexan gewaschen. Es wurden 14,5 kg Kristalle vom Smp. 48° erhalten.

Beispiel 5

Herstellung von

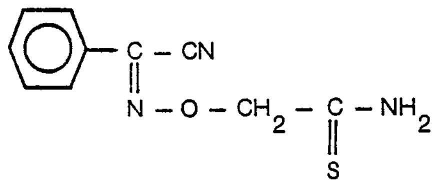

40 g (0,02 Mol) der Verbindung

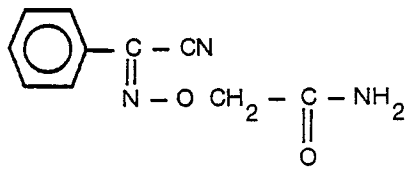

wurden
in 20 ml Hexamethylphosphorsäure-triamid gelöst und mit 4,0 gr (0,01 M) dimerem p-Methoxyphenylthionophosphinsulfid versetzt. Diese Suspension wurde für 30 Min. auf 80°C aufgeheizt. Nach dem Abkühlen wurde das Reaktionsgemisch auf 50 ml Wasser gegossen und das abgeschiedene Oel mit Aether extrahiert. Der Extrakt wurde getrocknet und eingedampft. Der Rückstand wurde mit Aether über eine kleine Kieselgelsäule filtriert. Nach dem Eindampfen des Filtrats wurden 3,4 gr hellbrauner Kristalle vom Smp. 104—106°C erhalten.

Beispiel 6

Herstellung von

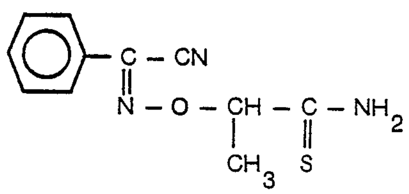

7,6 gr (0,05 M) 2-Brompropionsäureamid wurden in 30 ml Hexametapol gelöst und bei Raumtemperatur mit 10,1 gr (0,025 M) dimerem p-Methoxyphenylthionophosphin-sulfid versetzt. Durch leicht exotherme Reaktion stieg die Temperatur auf 33°C. Es wurde 30 Min. bei 80°C ausgerührt und das Reaktionsgemisch auf 40°C abgekühlt. Zu der klaren gelben Lösung wurden 9,9 gr (0,05 M) des Natriumsalzes von $\alpha$-Phenylacetonitriloxim (85%), das noch ca. 15% Isopropanol enthielt, gegeben. Durch exotherme Reaktion stieg die Temperatur auf 52°C. Die entstandene gelbe Suspension wurde bei 45°C noch 10 Min. ausgerührt. Das Reaktionsgemisch wurde auf 100 ml Wasser gegossen und mit Methylen-chlorid extrahiert. Nach dem Trocknen der organischen Phase wurde diese am Rotavap eingedampft und den Rückstand über eine Kieselgelsäule mit Petroläther/Aether 5:1 chromatographiert. Das Eindempfen der Hauptfraktion ergab 7,3 gr (62,9%) gelbe Kristalle vom Smp. 85—90°.

Auf analoge Weise oder nach einer der hierin beschriebenen Methoden können folgende Verbindungen der Formel I hergestellt werden.

TABELLE I

Ar 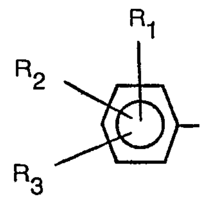

| Verb. Nr. | $R_1$ | $R_2$ | $R_3$ | m | n | X | Q | physik. Daten |
|---|---|---|---|---|---|---|---|---|
| 1 | H | H | H | 0 | – | CN | $-CH_2COONa$ | |
| 2 | H | H | H | 0 | – | CN | $-CH_2COOH$ | Smp. 169–171° |
| 3 | H | H | H | 0 | – | CN | $-CH_2-CH(OCH_3)_2$ | Smp. 35° |
| 4 | H | H | H | 0 | – | CN | $-CH_2COC_6H_5$ | Smp. 80° |
| 5 | H | H | H | 0 | – | CN | 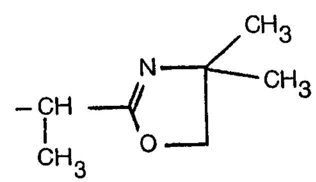 | Smp. 48° |
| 6 | H | H | H | 0 | – | CN | $-CH_2CSNH_2$ | Smp. 104–106° |
| 7 | H | H | H | 0 | – | CN | 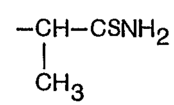 | Smp. 85–90° |
| 8 | H | H | H | 0 | – | CN | 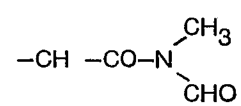 | Smp. 83° |
| 9 | H | H | H | 0 | – | CN | 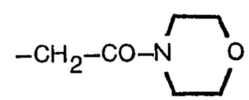 | Smp. 114–116° |

TABELLE I (fortsetzung)

| Verb. Nr. | $R_1$ | $R_2$ | $R_3$ | m | n | X | Q | physik.Daten |
|---|---|---|---|---|---|---|---|---|
| 10 | H | H | H | 0 | – | CN | $-CH_2CONHCOOCH_3$ | Smp. 137–138° |
| 11 | H | H | H | 0 | – | CN | $-CH_2CONHCONH_2$ | Smp. 182–184° |
| 12 | H | H | H | 0 | – | CN | $-CH_2CONHCOOC_2H_5$ | |
| 13 | 2–Cl | 4–Cl | H | 0 | – | CN | $-CH_2CONHCONH_2$ | Smp. 179–182° |
| 14 | 2–Cl | 4–Cl | H | 0 | – | CN | $-CH_2CONHCOOCH_3$ | |
| 15 | 2–Cl | 4–Cl | H | 0 | – | CN | $-CH_2CON(CH_3)CHO$ | zahfl. Oel |
| 16 | H | H | H | 1 | 2 | CN | $-CH_2CON(CH_3)CHO$ | Smp. 127–130° |
| 17 | H | H | H | 1 | 2 | CN | $-CH_2CONHCONH_2$ | Smp. 160–163° |
| 18 | H | 4–$CH_3$ | H | 1 | 2 | CN | $-CH_2CONHCOOCH_3$ | Smp. 167–168° |
| 19 | H | H | H | 0 | – | CN | $-CH_2CONHNH_2$ | Smp. 129–131° |
| 20 | H | H | H | 0 | – | CN | $-CH(CH_3)CONHNH_2$ | Smp. 116–119° |
| 21 | H | 4–Cl | H | 0 | – | CN | $-CH_2CONHNH_2$ | SMp. 139–141° |
| 22 | H | 4–Cl | H | 0 | – | CN | $-CH(CH_3)CONHNH_2$ | Smp. 162–163° |

TABELLE I (fortsetzung)

| Verb. Nr. | $R_1$ | $R_2$ | $R_3$ | m | n | X | Q | physik. Daten |
|---|---|---|---|---|---|---|---|---|
| 23 | 4–$CH_3$ | H | H | 0 | – | CN | $-CH(CH_3)CONHNH_2$ | |
| 24 | 2–Cl | 4–Cl | H | 0 | – | CN | $-CH_2CONHNH_2$ | |
| 25 | 2–Cl | 4–Cl | H | 0 | – | CN | $-CH(CH_3)CONHNH_2$ | |
| 26 | H | H | H | 0 | – | CN | $-CH_2COON(C_4H_9)_4$ | |
| 27 | H | H | H | 0 | – | CN | $-CH_2CO$<br>$ON(CH_2CH_2OH_2)$ | |
| 28 | H | 3–Cl | H | 0 | – | $CH_3$ | $-CH_2COOH$ | Smp. 93–95° |
| 29 | H | 4–Cl | H | 0 | – | $CH_3$ | $-CH_2COOH$ | Smp. 117–120° |
| 30 | H | 4–$CH_3$ | H | 0 | – | $CH_3$ | $-CH_2COOH$ | Smp. 114–116° |
| 31 | 3–$CH_3$ | 4–Cl | H | 0 | – | $CH_3$ | $-CH_2COOH$ | Smp. 97–101 |
| 32 | 2–Cl | 4–Cl | H | 0 | – | $CH_3$ | $-CH_2COOH$ | Smp. 86–88° |
| 33 | H | 4–Cl | H | 0 | – | CN | $-CH_2CH_2COOH$ | |
| 34 | H | 4–Cl | H | 0 | – | $CH_3$ | $-CH_2CH_2COOH$ | Smp. 80–83° |
| 35 | H | 4–Cl | H | 0 | – | $CH_3$ | $-CH_2CH_2CH_2$<br>$COOH$ | Smp. 106–8° |
| 36 | H | 4–Cl | H | 0 | – | $CH_3$ | $-CH-COOH$<br>$CH_3$ | Smp. 87–89° |

TABELLE I (fortsetzung)

| Verb. Nr. | $R_1$ | $R_2$ | $R_3$ | m | n | X | Q | physik. Daten |
|---|---|---|---|---|---|---|---|---|
| 37 | H | H | H | 0 | – | CN | 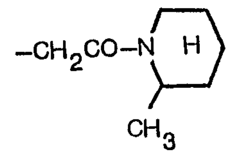 | Oel |
| 38 | H | 4–$CH_3$ | H | 1 | 2 | CN | 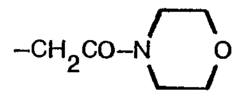 | Smp. 142–145° |
| 39 | H | H | H | 0 | – | CN | $-CH_2COCH_3$ | Smp. 68° |
| 40 | H | H | H | 0 | – | CN | 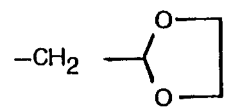 | Smp. 67° |
| 41 | H | H | H | 0 | – | CN | 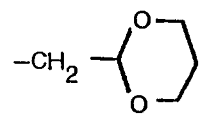 | Smp. 52–53° |
| 42 | H | H | H | 0 | – | CN | 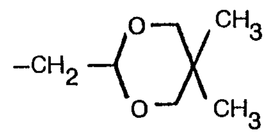 | Smp. ~ 30° |
| 43 | H | H | H | 0 | – | CN | 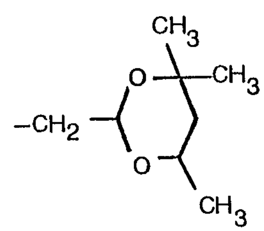 | Smp. 60° |

TABELLE I (fortsetzung)

| Verb. Nr. | $R_1$ | $R_2$ | $R_3$ | m | n | X | Q | physik. Daten |
|---|---|---|---|---|---|---|---|---|
| 44 | H | H | H | 0 | – | CN | $-CH_2CH(OC_2H_5)_2$ | $n_D^{21}$ = 1,5110 |
| 45 | H | $4-CH_3$ | H | 0 | – | CN | 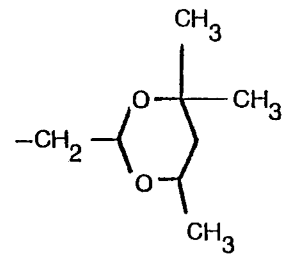 | Smp. 97° |
| 46 | H | 2–Cl | H | 0 | – | CN | 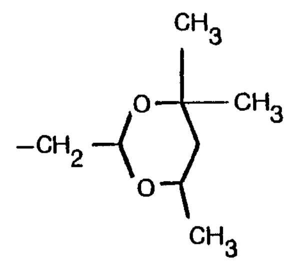 | $n_D^{24}$ = 1,5170 |
| 47 | H | 4–Cl | H | 0 | – | $CH_3$ | 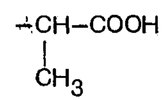 | Smp. 84–85° |
| 48 | H | $4-CH_3O$ | H | 0 | – | CN | 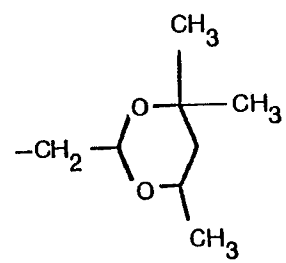 | $n_D^{24}$ = 1,5281 |

TABELLE I (fortsetzung)

| Verb. Nr. | $R_1$ | $R_2$ | $R_3$ | m | n | X | Q | physik. Daten |
|---|---|---|---|---|---|---|---|---|
| 49 | H | 2–Cl | H | 0 | – | CN | $-CH_2-CH(OCH_3)_2$ | $n_D^{24} = 1,5304$ |
| 50 | H | $4-CH_3$ | H | 0 | – | CN | $-CH_2-CH(OCH_3)_2$ | $n_D^{24} = 1,5258$ |
| 51 | H | $4-CH_3O$ | H | 0 | – | CN | $-CH_2-CH(OCH_3)_2$ | $n_D^{24} = 1,5375$ |
| 52 | 3–Cl | 4–Cl | H | 0 | – | CN | 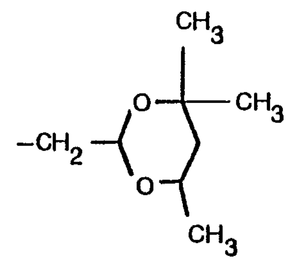 | $n_D^{24} = 1,5354$ |
| 53 | 3–Cl | 4–Cl | H | 0 | – | CN | $-CH_2-CH(OCH_3)_2$ | $n_D^{22} = 1,5523$ |
| 54 | 3–Cl | 4–Cl | H | 0 | – | CN | 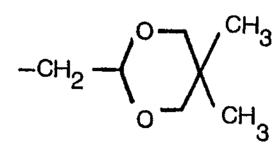 | $n_D^{22} = 1,5355$ |
| 55 | H | 2–Cl | H | 0 | – | CN | 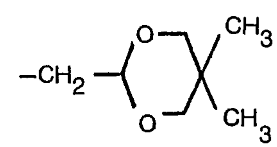 | $n_D^{22} = 1,5236$ |
| 56 | H | $4-CH_3O$ | H | 0 | – | CN | 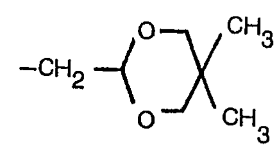 | Smp. 85° |

TABELLE I (fortsetzung)

| Verb. Nr. | $R_1$ | $R_2$ | $R_3$ | m | n | X | Q | physik. Daten |
|---|---|---|---|---|---|---|---|---|
| 57 | H | 4–CH | H | 0 | – | CN | 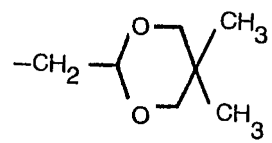 | Smp. 75° |
| 58 | H | $4-CH_3O$ | H | 0 | – | CN | 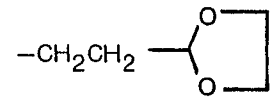 | $n_D^{21} = 1,5437$ |
| 59 | H | 2–Cl | H | 0 | – | CN | 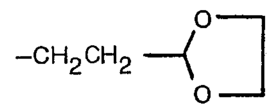 | $n_D^{24,5} = 1,5435$ |
| 60 | 3–Cl | 4–Cl | H | 0 | – | CN | 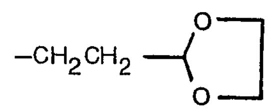 | Wachs |
| 61 | H | H | H | 0 | – | CN | 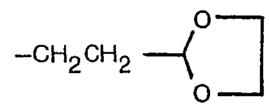 | $n_D^{24} = 1,5443$ |
| 62 | H | 4–CN | H | 0 | – | $COOCH_3$ | $CH_2CONHNH_2$ | |
| 63 | H | 4–CN | H | 0 | – | $CH_3$ | $CH_2COOH$ | |
| 64 | H | 4–CN | H | 0 | – | CN | $CH_2CH_2CONHNH_2$ | |
| 65 | H | 4–CN | H | 0 | – | CN | 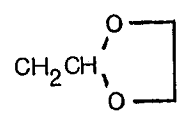 | |

TABELLE I (fortsetzung)

| Verb. Nr. | $R_1$ | $R_2$ | $R_3$ | m | n | X | Q | physik. Daten |
|---|---|---|---|---|---|---|---|---|
| 66 | H | 2–CN | H | 0 | – | CN | $CH_2CONHCOC_6H_5$ | |
| 67 | H | 4–$NO_2$ | H | 0 | – | $CH_3$ | $CH_2CONHNH_2$ | |
| 68 | H | 4–$NO_2$ | H | 0 | – | CN | 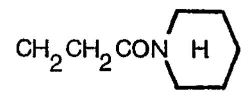 | |
| 69 | H | 2–$CH_3$ | 4–OCO<br>NH<br>$CH_3$ | 0 | – | $CH_3$ | CH–$CONC_6H_5$<br>$CH_3$ $NH_2O$ | |
| 70 | 3–OCH | 2–NO | 4–OCO<br>$CH_3$ | 0 | – | CN | $CH(CH_3)COO^{\ominus\oplus}NH_4$ | |
| 71 | 3–Cl | 2–$OCOCH_3$ | 5–Cl | 0 | – | $CH_3$ | 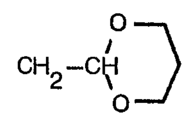 | |
| 72 | 4–$CH_3$ | 2–$OCOCH_3$ | 6–$CH_3$ | 0 | – | H | $CH(CH_3)COO^{\ominus}$ $^{\oplus}Na$ | |
| 73 | H | 4–$N(C_2H_5)_2$ | H | 0 | – | $CH_3$ | 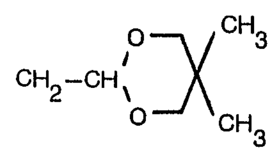 | |
| 74 | 3–$CH_3$ | 5–$CH_3$ | H | 0 | – | $CH_3$ | $CH_2$–$COCH_3$ | |

TABELLE I (fortsetzung)

| Verb. Nr. | $R_1$ | $R_2$ | $R_3$ | m | n | X | Q | physik. Daten |
|---|---|---|---|---|---|---|---|---|
| 75 | H | 4-Cyclohexyl | H | 0 | – | H | $CH_2–CO–C_6H_5$ | |
| 76 | H | 4–Br | H | 0 | – | $CONHCH_3$ | $CH_2CONHNH_2$ | |
| 77 | H | $2–OCH_2–C_6H_5$ | H | 0 | – | $CH_3$ | $CH_2CON(CH_3)COCH_2Cl$ | |
| 78 | H | $4–C_6H_4Br(4)$ | H | 0 | – | $CH_3$ | 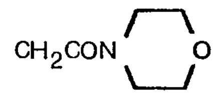 | |
| 79 | H | $4–OCH_2C_6H_5$ | H | 0 | – | $CH_3$ | 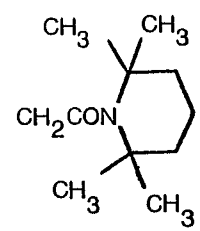 | |
| 80 | H | $4–CH{=}CH–C_6H_5$ | H | 0 | – | $CH_3$ | $CH_2COCH_3$ | |
| 81 | 3–Cl | $2–OCOCH_3$ | 6–Cl | 0 | – | $C_2H_5$ | $CH_2–CH(OCH_3)_2$ | |
| 82 | 3–Cl | $2–OCOCH_3$ | 6–Cl | 0 | – | $CH_3$ | $CH_2–CH(OC_2H_5)_2$ | |
| 83 | 5–Cl | $2–OCONHCH_3$ | H | 0 | – | $CH_3$ | $CH_2CH_2CH_2CONHNH_2$ | |
| 84 | 2–Cl | H | H | 0 | – | CN | 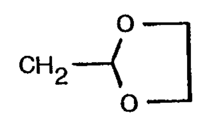 | Fp. 39° |
| 85 | H | H | H | 0 | – | CN | 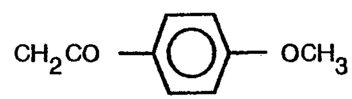 | Fp. 96–8° |

TABELLE I (fortsetzung)

| Verb. Nr. | $R_1$ | $R_2$ | $R_3$ | m | n | X | Q | physik. Daten |
|---|---|---|---|---|---|---|---|---|
| 86 | H | 4–$OCH_3$ | H | 0 | – | CN | $CH_2CH(OC_2H_5)_2$ | $n_D^{22}$ = 1,5250 |
| 87 | 2–Cl | 4–Cl | H | 0 | – | CN | $CH_2CH(OC_2H_5)$ | $n_D^{22}$ = 1,5237 |
| 88 | H | H | H | 0 | – | CN | $C_2H_4CH(OC_2H_5)_2$ | $n_D^{20}$ = 1,4947 |
| 89 | 2,3–$C_4H_4$– | | H | 0 | – | CN | $CH_2CH(OC_2H_5)_2$ | $n_D^{20}$ = 1,5462 |
| 90 | 4–$CH_3$ | H | H | 0 | – | CN | 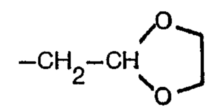 | Smp. 55° |
| 91 | 4–$OCH_3$ | H | H | 0 | – | CN | 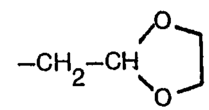 | Smp. 55° |
| 92 | 4–Cl | 3–Cl | H | 0 | – | CN | 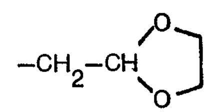 | Smp. 65° |
| 93 | 4–$CH_3$ | H | H | 0 | – | CN | 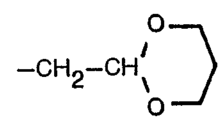 | Smp. 59° |
| 94 | 4–$OCH_3$ | H | H | 0 | – | CN | 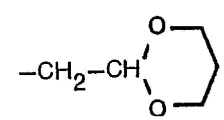 | Smp. 59° |
| 95 | 2–Cl | H | H | 0 | – | CN | 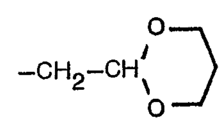 | |

TABELLE I (fortsetzung)

| Verb. Nr. | $R_1$ | $R_2$ | $R_3$ | m | n | X | Q | physik. Daten |
|---|---|---|---|---|---|---|---|---|
| 96 | 4–Cl | 3–Cl | H | 0 | – | CN | 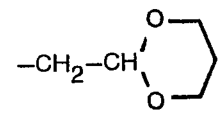 | |
| 97 | $4-OCH_3$ | H | H | 0 | – | CN | 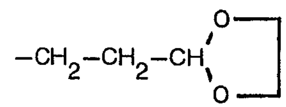 | $n_D^{24} = 1{,}5492$ |
| 98 | H | H | H | 0 | – | CN | 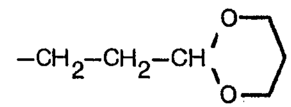 | $n_D^{23,5} = 1{,}5420$ |
| 99 | H | H | H | 0 | – | CN | 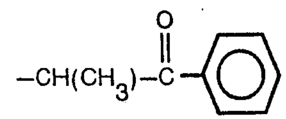 | 85° |
| 100 | H | H | H | 0 | – | CN | 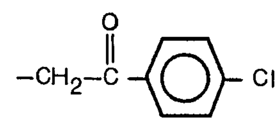 | Smp. 97° |
| 101 | H | H | H | 0 | – | CN | 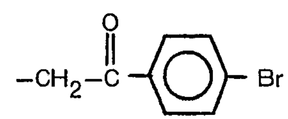 | Smp. 111° |
| 102 | $4-CH_3$ | H | H | 0 | – | CN | $-CH_2-CH(OC_2H_5)_2$ | $n_D^{21,5} = 1{,}5149$ |
| 103 | 4–Cl | 3–Cl | H | 0 | – | CN | $-CH_2-CH(OC_2H_5)_2$ | $n_D^{22} = 1{,}5303$ |

TABELLE I (fortsetzung)

| Verb. Nr. | $R_1$ | $R_2$ | $R_3$ | m | n | X | Q | physik. Daten |
|---|---|---|---|---|---|---|---|---|
| 104 | 4–Cl | H | H | 0 | – | CN | $-CH_2-CH(OC_2H_5)_2$ | $n_D^{20}$ = 1,5190 |
| 105 | $4-CH_3$ | H | H | 0 | – | CN | $-CH_2-CH_2-CH(OC_2H_5)_2$ | $n_D^{24}$ = 1,5008 |
| 106 | $4-OCH_3$ | H | H | 0 | – | CN | $-CH_2-CH_2-CH(OC_2H_5)_2$ | $n_D^{24}$ = 1,5114 |
| 107 | 4–Cl | 3–Cl | H | 0 | – | CN | $-CH_2-CH_2-CH(OC_2H_5)_2$ | $n_D^{24}$ = 1,5220 |
| 108 | 4–Cl | 2–Cl | H | 0 | – | CN | $-CH_2-CH_2-CH(OC_2H_5)_2$ | $n_D^{24}$ = 1,5413 |
| 109 | 4–Cl | H | H | 0 | – | CN | $-CH_2-CH_2-CH(OC_2H_5)_2$ | $n_D^{24}$ = 1,5103 |
| 110 | $2,3-C_4H_4-$ | | H | 0 | – | CN | $-CH_2-CH_2-CH(OC_2H_5)_2$ | $n_D^{24}$ = 1,5400 |
| 111 | 4–Cl | 2–Cl | H | 0 | – | CN | 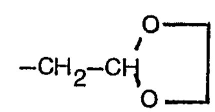 | $n_D^{24}$ = 1,5475 |
| 112 | 4–Cl | H | H | 0 | – | CN | 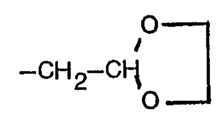 | $n_D^{24}$ = 1,5493 |
| 113 | $2,3-C_4H_4-$ | | H | 0 | – | CN | 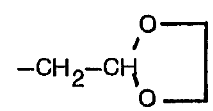 | $n_D^{24}$ = 1,5842 |

TABELLE I (fortsetzung)

| Verb. Nr. | $R_1$ | $R_2$ | $R_3$ | m | n | X | Q | physik. Daten |
|---|---|---|---|---|---|---|---|---|
| 114 | 4-Cl | 2-Cl | H | 0 | – | CN | $-CH_2-CH(OCH_3)_2$ | $n_D^{24}$ = 1,5428 |
| 115 | 2,3-$C_4H_4$- | | H | 0 | – | CN | $-CH_2-CH(OCH_3)_2$ | $n_D^{24}$ = 1,5707 |
| 116 | 4-Cl | 2-Cl | H | 0 | – | CN | 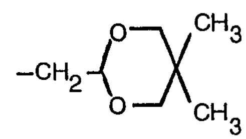 | $n_D^{24}$ = 1,5377 |
| 117 | 4-$CH_3$ | H | H | 0 | – | CN | 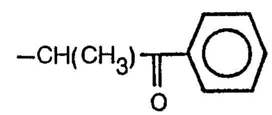 | $n_D^{22}$ = 1,5826 |
| 118 | 4-$OCH_3$ | H | H | 0 | – | CN | 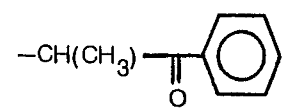 | Smp. 64–65° |
| 119 | 4-Cl | 3-Cl | H | 0 | – | CN | 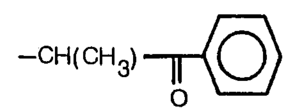 | $n_D^{22}$ = 1,5995 |
| 120 | H | H | H | 0 | – | CN | 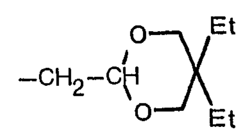 | $n_D^{22}$ = 1,5266 |
| 121 | 4-$CH_3$ | H | H | 0 | – | CN | 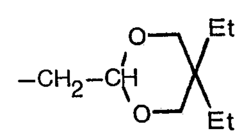 | Smp. 118–120° |

TABELLE I (fortsetzung)

| Verb. Nr. | $R_1$ | $R_2$ | $R_3$ | m | n | X | Q | physik. Daten |
|---|---|---|---|---|---|---|---|---|
| 122 | 4–Cl | 3–Cl | H | 0 | – | CN | 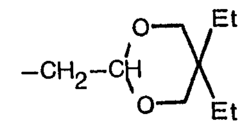 | Smp. 88–95° |
| 123 | 3–$CF_3$ | H | H | 0 | – | CN | $-CH_2CON(CH_3)CHO$ | |
| 124 | 3–$CF_3$ | H | H | 0 | – | CN | $-CH_2CONHNH_2$ | |
| 125 | 3–$CF_3$ | H | H | 0 | – | CN | $-CH_2CONHCOOCH_3$ | |
| 126 | 3–$CF_3$ | H | H | 0 | – | CN | $-CH_2CONHCONH_2$ | |
| 127 | 3–$CF_3$ | H | H | 0 | – | CN | 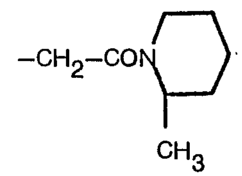 | |
| 128 | 3–$CF_3$ | H | H | 0 | – | CN | $-CH_2CH(OCH_3)_2$ | |
| 129 | 3–$CF_3$ | H | H | 0 | – | CN | $-CH_2CH(OC_2H_5)_2$ | |
| 130 | 3–$CF_3$ | H | H | 0 | – | CN | 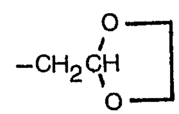 | |
| 131 | 3–$CF_3$ | H | H | 0 | – | CN | 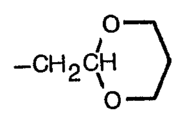 | |

TABELLE I (fortsetzung)

| Verb. Nr. | $R_1$ | $R_2$ | $R_3$ | m | n | X | Q | physik. Daten |
|---|---|---|---|---|---|---|---|---|
| 132 | $3-CF_3$ | H | H | 0 | – | CN | 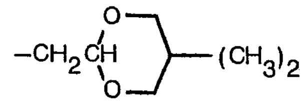 | |
| 133 | 3–Cl | H | H | 0 | – | CN | $-CH_2CH(OCH_3)_2$ | |
| 134 | 3–Cl | H | H | 0 | – | CN | $-CH_2CH(OC_2H_5)_2$ | |
| 135 | 3–Cl | H | H | 0 | – | CN | 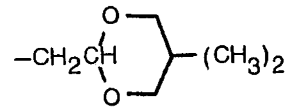 | |
| 136 | $3-CH_3$ | H | H | 0 | – | CN | 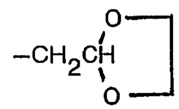 | |
| 137 | $3-OCH_3$ | H | H | 0 | – | CN | $-CH_2CH(OCH_3)_3$ | |
| 138 | $3-CH_3$ | H | H | 0 | – | CN | $-CH(OCH_2)-$ | |
| 139 | $3-CH_3$ | H | H | 0 | – | CN | $-CH(OC_2H_5)_2$ | |
| 140 | $3-OCH_2$ | H | H | 0 | – | CN | 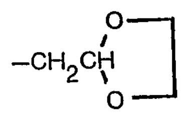 | |

TABELLE II

(Ar = 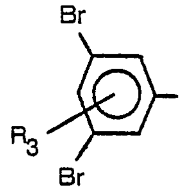 ; X = H, m = 0)

| Verb. Nr. | $R_3$ | Q |
|---|---|---|
| 141 | 4–$OCONHCH_3$ | $CH_2CONHNH_2$ |
| 142 | 4–$OCONHCH_3$ | $CH_2$–$CH(OCH_3)_2$ |
| 143 | 4–$OCONHCH_3$ | $CH_2$–$CH(OC_2H_5)_2$ |
| 144 | 4–$OCONHCH_3$ | 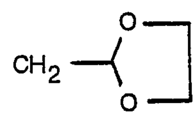 |
| 145 | 4–$OCONHCH_3$ | 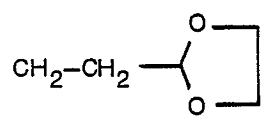 |
| 146 | 4–$OCOCH_3$ | $CH_2CONHNH_2$ |
| 147 | 4–$OCOCH_3$ | $CH_2$–$CH(OCH_3)_2$ |
| 148 | 4–$OCOCH_3$ | 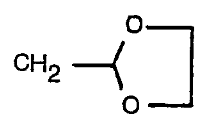 |
| 149 | 4–$OCONHC_2H_5$ | 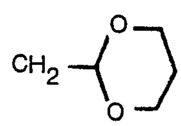 |
| 150 | 4–$OCONHC_6H_5$ | 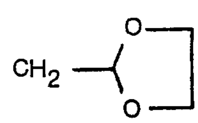 |
| 151 | 4–$OCONHC_6H_5$ | 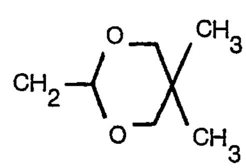 |
| 152 | 4–$OCONHC_2H_5$ | $CH_2CH(OC_2H_5)_2$ |
| 153 | 2–$OCH_2CH{=}CH_2$ | $CH_2CONHNH_2$ |

TABELLE II (fortsetzung)

| Verb. Nr. | $R_3$ | Q |
|---|---|---|
| 154 | $2\text{–}OCH_2CH{=}CH_2$ | 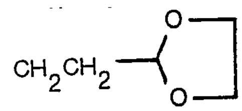 |
| 155 | $2\text{–}OCH_2CH{=}CH_2$ | 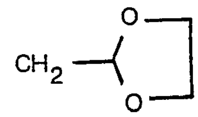 |
| 156 | $4\text{–}OCH_2CH{=}CH_2$ | 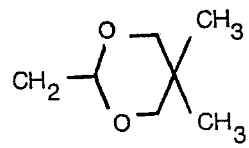 |
| 157 | $4\text{–}OCH_2\text{–}C{\equiv}CH$ | 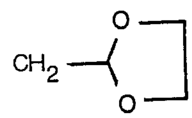 |
| 158 | $4\text{–}OCH_2\text{–}C{\equiv}CH$ | $CH_2CH_2CONHNH_2$ |
| 159 | $2\text{–}O\text{–}CH_2\text{–}C{\equiv}CH$ | 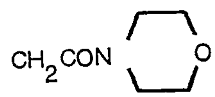 |
| 160 | $4\text{–}OCH_2CH{=}CH_2$ | 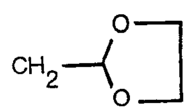 |
| 161 | $4\text{–}OCH_2CH{=}CH_2$ | 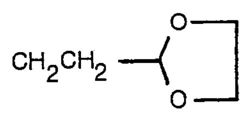 |
| 162 | $4\text{–}OCH_2CH{=}CH_2$ | $CH_2CH(C_2H_5)_2$ |

TABELLE III

( Ar = 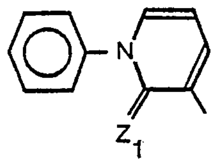 ; X = H ; m = o)

| Verb. Nr. | Y | Q |
|---|---|---|
| 163 | S | $-COOH$ |
| 164 | S | $-CH_2CSNH_2$ |
| 165 | S | $-CH_2CSN(CH_3)_2$ |
| 166 | S | $CH_2-CONHNH_2$ |
| 167 | O | 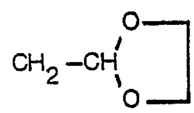 |
| 168 | O | 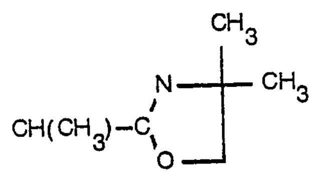 |
| 169 | O | 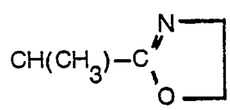 |
| 170 | O | $CH_2CONHNH_2$ |

TABELLE IV

( Ar = 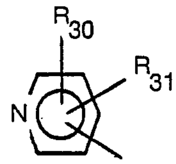 ; m = o)

| Verb. Nr. | $R_{30}$ | $R_{31}$ | $-CX=NOQ$ |
|---|---|---|---|
| 171 | 2–Cl | 6–Cl | $4-CH=NO-CH_2-CSNH_2$ |
| 172 | H | H | $4-CH=NO-CH_2$ 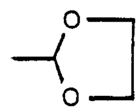 |
| 173 | H | H | $4-C(CH_3)=NOCH_2COC_6H_5$ |
| 174 | H | H | $2-CH=NO-CH(CH_3)$ 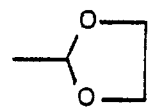 |
| 175 | H | H | $3-C(CH_3)=NO-CH_2CONHNH_2$ |
| 176 | H | H | $4-C(CN)=NO-CH_2CONHNH_2$ |
| 177 | H | H | $3-C(CH_3)=NO-CH_2CON(C_6H_5)NH_2$ |
| 178 | 2–Cl | 6–Cl | $3-C(CN)=NO-CH_2$ 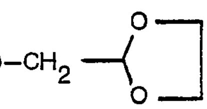 |

TABELLE V

( Ar = 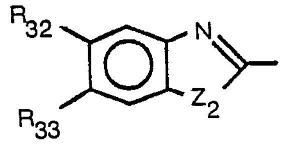 ; m = o)

| Verb. Nr. | $Z_2$ | $R_{32}$ | $R_{33}$ | X | Q |
|---|---|---|---|---|---|
| 179 | O | H | H | CN | $CH_2-CH(OCH_3)_2$ |
| 180 | O | H | H | CN | $CH_2-CSNH_2$ |
| 181 | O | H | H | H | $CH_2-CONHNH_2$ |
| 182 | O | H | $CH_3$ | CN | $CH_2-CON$ 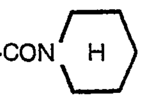 |
| 183 | O | Cl | H | CN | $CH(CH_3)COC_6H_5$ |
| 184 | S | H | H | CN | $CH_2-CH(OC_2H_5)_2$ |
| 185 | S | H | H | Cl | $CH_2CON$ 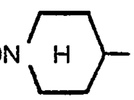 $-CH_3$ |
| 186 | S | H | H | CN | $CH(CH_3)CSNH_2$ |
| 187 | S | H | $CH_3$ | $CH_3$ | $CH_2CH_2CONHNH_2$ |
| 188 | S | H | $NO_2$ | $CH_3$ | $CH_2CH_2CONHNH_2$ |
| 189 | $NCH_3$ | H | H | CN | $CH_2$ 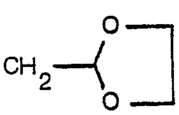 |
| 190 | $NCH_3$ | H | H | CN | $CH(CH_3)-COC_6H_5$ |
| 191 | $NCH_3$ | H | H | CN | $CH_2CH_2CH_2CH_2CONHNH_2$ |
| 192 | $NCH_3$ | $CH_3$ | H | CN | $CH_2-CSN(CH_3)_2$ |
| 193 | $NCH_3$ | Cl | H | $CH_3$ | $CH_2-CONCOC_6H_4Cl(4)$<br>\|<br>$C_2H_5$ |

TABELLE VI

( Ar = 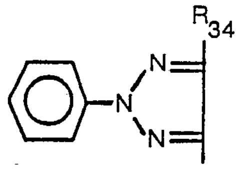 ; m = o ; X = H)

| Verb. Nr. | $R_{34}$ | Q |
|---|---|---|
| 194 | H | $CH_2CH(OCH_3)_2$ |
| 195 | $CH_3$ | $CH_2CH_2CONHNH_2$ |
| 196 | H | $(CH_2)_4CSN(CH_3)C_3H_7(n)$ |

TABELLE VII

( Ar = 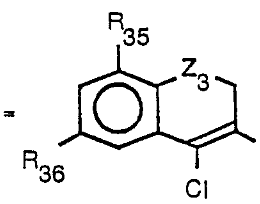 ; m = o ; X = O)

| Verb. Nr. | $Z_3$ | $R_{35}$ | $R_{36}$ | Q |
|---|---|---|---|---|
| 197 | O | H | Cl | $CH(CH_3)$ 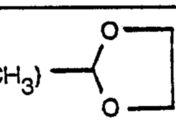 |
| 198 | O | H | Cl | $CH_2CON(C_6H_5)NH_2$ |
| 199 | O | Cl | H | $CH(C_2H_5)CON(CH_3)COC_3H_7(n)$ |
| 200 | O | Cl | Cl | $CH_2CH_2CSNHC_6H_5$ |
| 201 | O | H | H | $CH_2CH_2CSNHC_6H_3(CH_3)_2(2,4)$ |
| 202 | S | H | Cl | $CH(C_2H_5)CON(C_3H_{7(n)}$<br>$COC_3H_7n$ |
| 203 | S | Cl | Cl | $CH_2COOH$ |
| 204 | S | Cl | Cl | $CH_2COO^{\ominus}$ $^{\oplus}Na$ |

TABELLE VIII

( Ar = 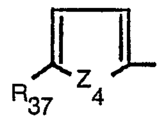 ; m = o)

| Verb. Nr. | $Z_4$ | $R_{37}$ | X | Q |
|---|---|---|---|---|
| 205 | S | H | CN | $CH(CH_3)-CSNHC_6H_3(CH_3)_2(2,6)$ |
| 206 | S | H | Cl | $CH_2CON$ 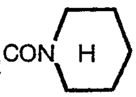 |
| 207 | S | H | CN | $CH_2CH_2CONHNH_2$ |
| 208 | S | H | CN | $CH_2CSNH_2$ |
| 209 | S | Cl | CN | $CH_2CONH_4H_9n$ $\vert$ $COC_6H_5$ |
| 210 | S | Cl | $CH_3$ | $CH(C_3H_7)CONHNH_2$ |
| 211 | O | Cl | CN | $CH_2CH_2CH_2CONHNH_2$ |
| 212 | O | Cl | $CH_3$ | $CH_2CH_2CH_2CSNH-C_6H_4Cl(2)$ |
| 213 | O | Cl | Cl | $CH_2-CON$ 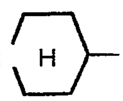 $CH_3$ |
| 214 | O | H | CN | $CH_2-CH$ 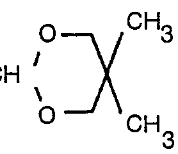 |
| 215 | O | H | CN | $CH_2-CSNH_2$ |
| 216 | O | H | $CH_3$ | $CH_2-CON$ 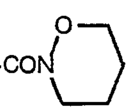 |
| 217 | O | H | $CH_3$ | $C(-CH_3)_2CONHNH_2$ |
| 218 | O | H | Cl | $CH_2-CON$ 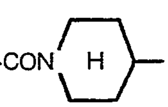 $CH_2$ |
| 219 | O | H | CN | $C(CH_3)-COCH_3$ |
| 220 | $NCH_3$ | H | CN | $CH_2CONHNH_2$ |
| 221 | $NCH_3$ | H | CN | $CH_2CH_2CONHNH_2$ |
| 222 | $NCH_3$ | H | $CH_3$ | $CH(CH_3)CON$ 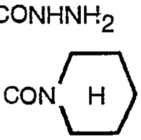 |
| 223 | S | Cl | $CH_3$ | $CH_2COOH$ Smp. 114–116° |

TABELLE IX

(Ar = 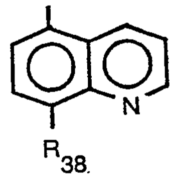 ; m = o ; X = $CH_3$)

| Verb. Nr. | $R_{38}$ | Q |
|---|---|---|
| 224 | $OCOCH_3$ | $CH_2CONHNH_2$ |
| 225 | $OCOCH_3$ | $CH_2CO$ 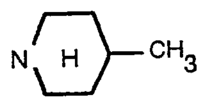 |
| 226 | $OCONHCH_3$ | $CH_2CON(CH_3)CO{-}C_6H_5$ |
| 227 | $OCOC_2H_5$ | $CH_2CH_2CH_2CON(C_6H_5)NH_2$ |
| 228 | $OCOCH_2Cl$ | $CH_2CH_2COOH$ |
| 229 | $OCONHC_2H_5$ | $CH_2$ 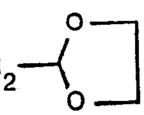 |
| 230 | $OCONHC_3H_7(i)$ | $CH(CH_3){-}C$ 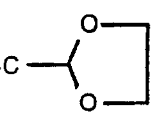 |
| 231 | $OCONHC_4H_9(n)$ | $CH(C_4H_9(n)CONHNH_2$ |

Formulierungsbeispiele

Beispiel 7

*Stäubemittel:*

Zur Herstellung eines a) 5%igen und b) 2%igen Stäubemittels werden die folgenden Stoffe verwendet;

a) 5 Teile Wirkstoff
95 Teile Talkum;

b) 2 Teile Wirkstoff
1 Teil hochdisperse Kieselsäure
97 Teile Talkum;

Die Wirkstoffe werden mit den Trägerstoffen vermischt und vermahlen und können in dieser Form zur Anwendung verstäubt werden.

Beispiel 8

*Granulat:*

Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft.

Beispiel 9

*Spritzpulver:*

Zur Herstellung eines a) 70%igen, b) 40%igen c) und d) 25%igen, e) 10%igen Spritzpulvers werden folgende Bestandteile verwendet:

a) 70 Teile Wirkstoff
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 40 Teile Wirkstoff
5 Teile Ligninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 25 Teile Wirkstoff
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkoholsulfaten,
5 Teile Naphthalinsulfonsäure/Formaldehyd-kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Behandlung von Pflanzenteilen verwenden lassen.

Beispiel 10

*Emulgierbare Konzentrate:*

Zur Herstellung eines 25%igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

Biologische Beispiele

Beispiel 11

Vorauflauf-Antidote-Test (Grundtest)

*Allgemeine Methodik:*

Kleine Blumentöpfe (oberer Ø 6 cm) werden mit Gartenerde gefüllt, in die die Pflanzenkultur eingesät, bedeckt und leicht festigedrückt wird. Dann wird die als Antidote zu prüfende Substanz als (aus einem Spritzpulver erhaltene) verdünnte Lösung in einer Menge aufgesprüht, die 4 kg AS/ha entspricht. Unmittelbar danach wird in entsprechender Weise das herbizid aufgesprüht. Nach 18 Tagen Stehen bei ca. 20 bis 23°C und 60 bis 70% relativer Luftfeuchtigkeit wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Es wurden verwendet:

1) 1,5 kg As/ha $\alpha$-[4-(p-Trifluormethylphenoxy)-phenoxy]-propionsäure-n-butoxyäthylester in *Mais* der Sorte "Orla 264".

2) 1,5 kg As/ha Metolachlor = N-(1-Methyl-2-methoxyäthyl)-N-chloracetyl-2-äthyl-6-methylanilin in *Sorghum-Hirse* der Sorte "Funk G-522".

3) 2,0 kg AS/ha Prometryn = 2,4-bis(Isopropylamino)-6-methylthio-s-triazin in *Soja*.

4) 2,0 kg AS/ha 4-Aethylamino-6-tert.butylamino-2-chlor-s-triazin in *Weizen* der Sorte "Farnese".

5) 4,0 kg AS/ha Prometryn = 2,4-bis(Isopropylamino)-6-methylthio-s-triazin in *Sorghum-Hirse* der Sorte "Funk G-522".

6) 2,0 kg AS/ha $\alpha$-[4-(p-Trifluormethylphenoxy)-phenoxy]-propionsäure-n-butoxyäthylester in *Gerste* der Sorte "Mazurka".

Verbindungen der Formel (I) erzielen in diesen Versuchen gute Antidote-Wirkung. Als Beispiele seien die folgenden genannt:

| Testvariante | Verbindung Nr. | Note des Herbizideinflusses (ohne/mit Antidote |
|---|---|---|
| 5 | 37 | 5 / 8 |
| 5 | 41 | 4 / 7 |
| 5 | 42 | 4 / 6 |
| 5 | 43 | 4 / 7 |
| 6 | 40 | 3 / 7 |
| 6 | 84 | 5 / 7 |

Beispiel 12

Antidote-Wirkung bei getrennter Applikation (Antidote-Vorauflauf. Herbizid-Nachauflauf)

*Allgemeine Methodik:*

Kleine Blumentöpfe (oberer Ø 6 cm) werden mit sandiger Lehmerde gefüllt, in die die Kulturpflanze eingesät wird. Nach dem Bedecken des Samens sprüht man die als Antidote zu prüfende Substanz in verdünnter Lösung und in einer Menge auf die Oberfläche, die umgerechnet 4 kg AS/ha beträgt. Man hält bei 20 bis 23°C und 60 bis 70% relativer Luftfeuchtigkeit. Wenn die Pflanzen nach 10 Tagen das 2- bis 3-Blattstadium erreicht haben, werden sie, wie nachfolgend angegeben, mit der entsprechenden Menge Herbizid behandelt. 14 Tage nach Herbizid-Applikation wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Man verwendet:

1) 4,0 kg AS/ha Ametryn = 2-Aethylamino-4-isopropylamino-6-methylthio-s-triazin in *Mais* der Sorte "Orla 264".

2) 1,0 kg SA/ha Prometryn = 2,4-bis (Isopropylamino)-6-methylthio-s-triazin in Sorghum-Hirse der Sorte "Funk G-522".

3) 0,25 kg As-ha $\alpha$[4-(p-Trifluormethylphenoxy)-phenoxy]-propionsäure-n-butoxyäthylester in *Gerste* der Sorte "Mazurka".

Verbindung der Formel (I) erzielten in diesen Versuchen eine gute Antidote-Wirkung.

Beispiel 13

Antidote-Wirkung an verpflanztem Reis bei getrennter Applikation (Antidote-Vorauflauf. Herbizid-Nachauflauf)

Plastiktöpfe (8 x 8 cm, 10 cm Höhe) werden bis 2 cm unter den Rand mit Erde im sumpfignassen Zustand gefüllt. Die als Antidote zu prüfende Substanz wird in verdünnter Lösung und in einer Menge auf die Oberfläche gesprüht, die 4 kg AS/ha entpsricht. Reispflanzen der Sorte "IR-8" werden im 1 1/2- bis 2-Blattstadium in die so vorbereiteten Töpfe verpflanzt. Am nächsten Tag wird der Wasserstand auf ca. 1,5 cm erhöht. 4 Tage nach Verpflanzung werden umgerechnet 0,75 kg AS/ha von 2-Aethylamino-4-(1,2-dimethyl-n-propylamino)-6-methylthio-s-triazin in Granulatform ins Wasser gegeben. Die Temperatur beträgt während der Versuchsdauer 26 bis 28°C, die relative Luftfeuchtigkeit 60 bis 80%. 20 Tage nach Herbizidbehandlung wird nach einer linearen Skala von 1 bis 9 ausgewertet, wobei 1 totale Pflanzenschädigung und 9 unbeeeinträchtigter Gesundheitszustand bedeuten. Als Kontrolle dienen Pflanzen ohne Antidote-Schutz.

Mit den Verbindungen der Formel (I) wurden gute Antidote Wirkungen erzielt.

| Verb. Nr. | Note des Herbizideinflusses (ohne/mit Antidote) |
|---|---|
| 40 | 2 / 4 |
| 3 | 2 / 6 |
| 41 | 4 / 7 |
| 42 | 4 / 8 |
| 10 | 5 / 8 |
| 58 | 5 / 7 |
| 60. | 3 / 6 |

Beispiel 14

Vorauflauf-Antidote-Test in Nährlösung

Es wird eine Hewitt-Nährlösung hergestellt, die die nachstehend angagebene Menge Herbizid sowie 10 ppm des zu prüfenden Antidotes enthält.

Man verwendet Kultursamen, der in der angegebenen Prüfkonzentration von dem eingesetzten Herbizid erwartungsgemäss geschädigt werden sollte und sät ihn in gekörntes Zonolith (= expandiertes Vermikulit), das sich in einem an der Unterseite durchlöcherten Plastik-Blumentopf (oberer Ø 6 cm) befindet. Dieser wird in einen zweiten durchsichtigen Plastik-Blumentopf (oberer Ø 7 cm) gestellt, in dem sich ca. 50 ml der mit Herbizid und Antidote vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewitt-Nährlösung auf 50 ml ergänzt. 3 Wochen nach Testbeginn wird nach einer linearen Skala von 1 bis 9 ausgewertet, wobei 1 totale Pflanzenschädigung und 9 unbeeinträchtiger Gesundheitszustand bedeuten. Die parallel angewandte Kontroll-Lösung enthält keinen Antidote-Zusatz.

Man verwendet:

1) 4 ppm Prometryn = 2,4-bis (Isopropylamino)-6-methylthio-s-triazin in *Sorghum-Hirse* der Sorte "Funk G-522".

2) 4 ppm 4-Aethylamino-6-tert.butylamino-2-chlor-s-triazin in *Weizen* der Sorte "Farnese".

3) 4 ppm $\alpha$-[4-(p-Trifluormethylphenoxy)-phenoxy]-propionsäure-n-butoxyäthylester in *Gerste* der Sorte "Mazurka".

4) 5 ppm Metolachlor = N-(1-Methyl-2-methoxy-äthyl)-N-chloracetyl-2-äthyl-6-methylanilin in *Sorghum-Hirse* der Sorte "Funk G-522".

Mit den Verbindungen der Formel (I) erzielt man eine gute Antidote-Wirkung.

Es wurden beispielsweise folgende Ergebnisse erzielt:

| Testvariante | Verb. Nr. | Note des Herbizideinflusses (ohne/mit Antidote) |
|---|---|---|
| 1 | 54 | 3 / 6 |
| 3 | 85 | 3 / 6 |
| 4 | 3 | 4 / 7 |
| 4 | 11 | 2 / 6 |
| 4 | 40 | 4 / 7 |
| 4 | 41 | 4 / 7 |
| 4 | 42 | 4 / 7 |
| 4 | 53 | 3 / 7 |
| 4 | 54 | 3 / 8 |
| 4 | 55 | 3 / 8 |

Beispiel 15

Vorauflauf-Antidote Test in Nährlösung (Reis)

Es wird eine Hewitt-Närhlösung hergestellt, die zusätzlich 10 ppm des zu prüfenden Antidotes enthält.

Reissamen der Sorte "IR-8" wird in inertes Füllmaterial (gekörntes Zonolith) gesät, das sich in einem an der Unterseite durchlöcherten Plastik-Blumentopf (oberer Ø 6 cm) befindet. Dieser wird in einen zweiten durchsichtigen Plastik-Blumentopf (oberer Ø 7 cm) gestellt, in dem sich ca. 50 ml der vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewitt-Nährlösung auf 50 ml ergänzt. Nach 15 Tagen werden die Reispflanzen im 2- bis 2 1/2-Blattstadium in rechteckige Plastiktöpfe (8 x 8 cm, 10 cm Höhe) verpflanzt, die mit 500 ml sumpfignasser Erde gefüllt sind. Am nächsten Tag wird der Wasserstand darin auf 1 bis 2 cm über Boden-Niveau erhöht. 4 Tage nach Verpflanzung gibt man das Herbizid 2-Aethylamino-4-(1,2-dimethyl-n-propylamino)-6-methylthio-s-triazin in Granulatform und in einer Menge zu, die umgerechnet 0,75 kg AS/ha beträgt. 3 Wochen nach Herbizidzugabe wird nach einer linearen Skala von 1 bis 9 unbeeinträchtigter Gesundheitszustant bedeuten. Die parallel angewandte Kontroll-Lösung enthält keinen Antidote-Zusatz.

In diesem Test erzielten Verbindungen der Formel (I) eine gute Antidote-Wirkung. Es wurde z.B. folgende Wirkung erzielt:

| Verb. Nr. | Herbizideinfluss ohne/mit Antidote |
|---|---|
| 21 | 1 / 7 |
| 87 | 2 / 6 |
| 86 | 5 / 6 |

Beispiel 16

Nachauflauf-Antidote-Test in Nährlösung

*Allgemeine Methodik:*

Man füllt kleine Plastik-Blumentöpfe (oberer Ø 6 cm), die an der Unterseite durchlöchert sind, mit gekörntem Zonolith und sät den Kultursamen ein. Dann wird der Topf in einen zweiten durchsichtigen Plastik-Blumentopf (oberer Ø 7 cm) gestellt, in dem sich 50 ml Wasser befinden, das kapillar aufsteigt und den Samen benetzt. Ab 5. Tag wird der laufende Wasserverlust mit Hewitt-Nährlösung ausgeglichen. Ab 15. Tag, wenn sich die Kulturpflanze im 1 1/2- bis 2-Blattstadium befindet, wird in die wieder auf 50 ml ergänzte Nährlösung.

10 ppm des zu prüfenden Antidotes + die unten angegebene Menge Herbizid

zugegeben. Ab 16. Tag wird der Flüssigkeitsverlust wieder durch reine Hewitt-Nährlösung ausgeglichen. Während der gesamten Testdauer beträgt die Temperatur 20 bis 23°C bei einer relativen Luftfeuchtigkeit von 60 bis 70%. 3 Wochen nach Zugabe des Herbizids und des Antidotes erfolgt die Auswertung nach einer linearen Skala von 1 bis 9, wobei 1 totale Pflanzenschädigung und 9 unbeinträchtigter Gesundheitszustand bedeuten.

*Testvarianten:*

1) 15 ppm α[4-(3,5-Dichlorpyridyl-2-oxy)-phenoxy]-propionsäure-propargylthioloester in *Weizen* der Sorte "Zenith".

2) 4 ppm 4-Aethylamino-6-tert.butylamino-2-chlor-s-triazin in *Weizen* der Sorte "Zenith".

3) 2 ppm α-[4-(p-Trifluormethylphenoxy)-phenoxy]-propionsäure-n-butoxyäthylester in *Mais* der Sorte "Orla".

4) 8 ppm α-[4-(p-Trifluormethylphenoxy)-phenoxy]-propionsäure-n-butoxyäthylester in *Sorghum-Hirse* der Sorte "Funk G-522".

5) 4 ppm Prometryn = 2,4-bis(Isopropylamino)-6-methylthio-s-triazin in *Sorghum-Hirse* der Sorte "Funk G-522".

6) 8 ppm α[4-(3,5-Dichlorpyridyl-2-oxy)-phenoxy]-propionsäuremethylester in *Weizen* der Sorte "Zenith".

Verbindungen der Formel (I) erzielen bei diesen Versuchen gute Antidote-Wirkung.

Beispiel 17

Antidote Test Samenquellung (Seed Soaking)

Reissamen der Sorte IR 8 werden während 48 Stunden mit Lösungen der Testsubstanzen von 10, 100 oder 1000 ppm getränkt. Anschliessend werden die Samen etwa 2 Stunden trocknen gelassen, bis sie nicht mehr kleben. Rechteckige Plastiköpfe (8 x 8 cm, 10 cm Höhe) werden bis 2 cm unter den Rand mit sandigem Lehm gefüllt. 4 g Samen werden pro Topf gesät und nur ganz schwach gedeckt (etwa Durchmesser des Samenkorns). Die Erde wird in einem feuchten (nicht sumpfigen) Zustand gehalten. Dann wird das Herbizid N-(1-Methyl-2-methoxyäthyl)-N-chloracetyl-2-äthyl-6-methylanilin in verdünnter Lösung und in einer Menge appliziert, die umgerechnet 1,5 kg AS/ha entspricht. 7 und 18 Tage nach dem Verpflanzen wird nach einer linearen Skala von 1 bis 9 ausgewertet, wobei 1 totale Pflanzenschädigung und 9 unbeeinträchtigter Gesundheitszustand bedeuten.

In diesem Test erzielten Verbindungen No. 3, 8, 10, 37, 40 und 50 eine gute Antidote-Wirkung.

Beispiel 18

Antidote-Test Wurzelbehandlung (Root dipping)

Reispflanzen der Sorte IR 8 werden bis zum 1 1/2 bis 2 Blattstadium in Erde herangezogen und dann flüchtig ausgewaschen. Die Pflanzen werden dann büschelweise nur mit den Wurzeln während 45 Minuten in eine Schale mit Lösungen der Testsubstanz von 10, 100 oder 1000 ppm getaucht. Anschliessend werden sie in Container (47 cm lang, 29 cm breit und 24 cm hoch) in sandigen Lehm verpflanzt. Die Bodenoberfläche wird mit Wasser von 1 1/2 bis 2 cm Höhe beschichtet. Einen Tag nach dem Verpflanzen wird das Herbizid N-n-Propoxyäthyl-N-chloracetyl-2,6-diäthylanilin in verdünnter Lösung direkt ins Wasser in einer Menge pipettiert, die umgerechnet 1,5 kg AS/ha entspricht. 7 und 18 Tage nach dem Verpflanzen wird nach einer linearen Skala von 1 bis 9 ausgewartet, wobei 1 totale Pflanzenschädigung und 9 unbeeinträchtiger Gesundheitszustand bedeuten.

In diesem Test erzielen Verbindungen der Formel (I) gute Antidote-Wirkungen.

Beispiel 19

In einem mit Sojabohnen bestellten Feld (Sorte Lee 68) wurden Parzellen von 2,5 × 10 m mit wässrigen Wirkstoffbrühen bespritzt, so dass Mengen von 0,5, 1 und 2 kg Wirkstoff pro Hektar aufgetragen wurden. Die Soja-Pflanzen befanden sich zum Applikationszeitpunkt im 8—10 Blatt-Stadium. Unbehandelte Parzellen dienten als Kontrolle.

Im Erntezeitpunkt, 15 Wochen nach der Applikation wurde für jede Parzelle die mittlere Wuchshöhe bestimmt und der Ertrag an geernteten Bohnen gewogen.

Bei Aufwandmengen von 1 und 2 kg der Verbindung No. 41 wurde eine gegenüber der Kontrolle um ca 15—20% geringere Wuchshöhe festgestellt, während der Ertrag um 17 bis 24% gestiegen war.

## Patentansprüche

1. Verfahren zum Schutz von Pflanzenkulturen vor der phytotoxischen Wirkung starker Herbizide mittels Oxim-Derivaten der Formel

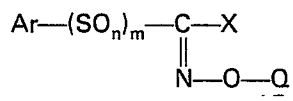

(I)

worin

n 0, 1 oder 2 und m 0 der 1 ist, und worin

Ar einen Phenylrest

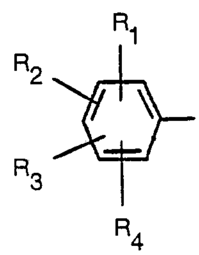

einen durch $R_2$ und $R_3$ substituierten Naphthylrest,

einen 5- bis 10-gliedrigen heterocyclischen Rest, der maximal 3 gleiche oder verschiedene Heteroatome N, O und/oder S enthält und durch $R_2$, $R_3$ und $R_4$ substituiert ist und durch Oxo oder Thiono substituiert sein kann, bedeutet, oder worin, wenn m = 0 ist,

Ar einen Rest R—CO— darstellt, worin

R einen Rest $—OR_5$ bedeutet, worin $R_5$ für eine aliphatische Gruppe mit maximal 8 C-Atomen oder araliphatische Gruppe mit maximal 15 C-Atomen oder eine cycloaliphatische oder aromatische Gruppe mit je maximal 10 C-Atomen steht, wobei als Substituenten der aromatischen Reste oder des cycloaliphatischen Restes Halogen, —CN, $—NO_2$, Niederalkyl, Niederalkoxy, Halogenalkyl in Frage kommen,

ein Rest $—NH—CO—NH—R_7$ oder

ein Rest $—N(R_6)\ (R_7)$ ist, worin $R_6$ für Wasserstoff oder Niederalkyl steht und $R_7$ Wasserstoff oder eine aliphatische Gruppe mit maximal 8 C-Atomen oder eine araliphatische Gruppe mit maximal 15 C-Atomen oder eine cycloaliphatische oder aromatische Gruppe mit je maximal 10 C-Atomen bedeutet, wobei als mögliche Substituenten der aromatischen Gruppe oder des cycloaliphatischen Restes Halogen, —CN, $NO_2$, Niederalkyl, Niederalkoxy, Halogenalkyl in Frage kommen,

einen Rest —$N(R_6)(R_7)$, wobei $R_6$ und $R_7$ zusammen mit dem N-Atom einen 5- oder 6-gliedrigen heterocyclischen Ring bilden, der als mögliches weiteres Heteroatom noch Sauerstoff enthalten kann,

$R_1$ Wasserstoff, Halogen, Niederalkyl, Niederalkoxy oder einen gegebenenfalls maximal zweimal durch Halogen, CN, $NO_2$, $CF_3$ substituierten paraständigen Phenoxyrest darstellt,

$R_2$, $R_3$, $R_4$ unabhängig voneinander Wasserstoff, Halogen, CN, $NO_2$, Niederalkyl, Niederalkoxy, Halogenalkyl, Halogenalkoxy, Niederalkanoyl, OH, Phenyl, Halogenphenyl, Niedercarbalkoxy, Niederalkoxycarbonyl, Niederalkoxycarbonyloxy, Niedercarbamoyloxy, Niederalkylthio, Niederalkylsulfonyl, Phenalkoxy, Cyclohexyl, $NH_2$, —NH-Niederalkyl, —N(Niederalkyl)$_2$, Niederalkanoylamino, Carbonsäureamid, Sulfonsäureamid bedeutet,

X für Wasserstoff, —CN, Halogen, Niederalkyl, Niederalkanoyl, —COOH, einen Carbonsäureesterrest, einen Carbonsäureamidrest steht, und

Q für den Rest —$C_aH_{2a}$—$R_8$ steht,

worin

a für eine ganze Zahl zwischen 1 und 6 steht, wobei der entsprechende Rest auch verzweigt sein kann, und $R_8$ für eine der nachfolgenden Gruppen steht.

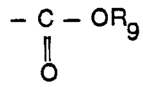 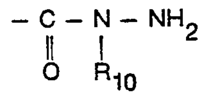 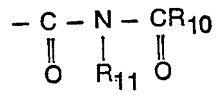 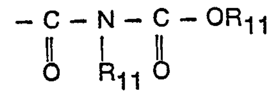

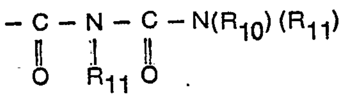 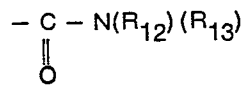 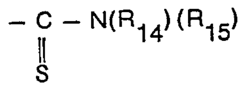

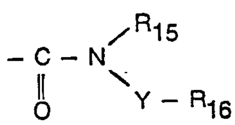 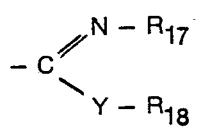 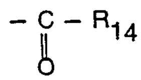 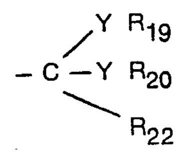

wobei die Substituenten $R_9$ bis $R_{20}$, $R_{22}$ und Y folgende Definitionen haben:

$R_9$ Wasserstoff oder Kation einer Base $^1/p\ M^{p\oplus}$ wobei

M eine Alkali- Erdalkali-Kation oder ein Fe-, Cu-, Zn-, Mn-, Co-, Ni-Kation oder einen Amino-Rest

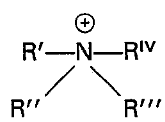

bedeuten, und

p als ganze Zahl 1, 2 oder 3 die Wertigkeit des Kations berücksichtigt, während R′, R″, R‴ und $R^{IV}$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten,

$R_{10}$ Wasserstoff, ein aliphatischer Rest, ein araliphatischer Rest oder ein gegebenenfalls ein- oder mehrfach durch Halogen, Halogenalkyl, Niederalkoxy und/oder Cyano substituierter aromatischer Rest

$R_{11}$ Wasserstoff, Niederalkyl

$R_{12}$ und $R_{13}$ zusammen mit dem Stickstoffatom ein gegebenenfalls ein- oder mehrfach durch Halogen, Cyano und/oder Niederalkyl substituierter und gegebenenfalls durch ein N, O oder S unterbrochener 5- bis 6-gliedriger Ring,

$R_{14}$ ein cycloaliphatischer oder ein araliphatischer oder aliphatischer Rest oder ein gegebenenfalls ein- oder mehrfach durch Halogen, Halogenalkyl, Niederalkoxy und/oder Cyano substituierter aromatischer Rest,

$R_{15}$ Wasserstoff, Niederalkyl oder Cycloalkyl,

$R_{16}$ ein gegebenenfalls durch Cyano substituierter aliphatischer Rest oder ein gegebenenfalls ein- oder mehrfach durch Halogen, Halogenalkyl, Niederalkoxy und/oder Cyano substituierter aromatischer Rest

$R_{17}$ Wasserstoff gegebenenfalls durch Halogen substituiertes Niederalkenyl oder Niederalkinyl, Niederalkyl oder Cycloalkyl,

$R_{18}$ Niederalkyl oder Cycloalkyl,

$R_{17}$ und $R_{18}$ zusammen mit —N=C—Y— ein gegebenenfalls durch Niederalkyl substituierter, 5- bis 6-gliedriger Ring,

$R_{19}$ und $R_{20}$ unabhängig voneinander Niederalkyl oder zusammen mit —Y—C—Y— ein gegebenenfalls durch Niederalkyl, Halogen und/oder Nitro substituierter 5- bis 6-gliedriger Ring,

$R_{22}$ Wasserstoff, ein cycloaliphatischer oder ein araliphatischer oder aliphatischer Rest oder ein

gegebenenfalls ein- oder mehrfach durch Halogen, Halogenalkyl, Niederalkoxy und/oder Cyano substituierter aromatischer Rest,

Y Sauerstoff oder Schwefel ist.

2. Ein herbizides Mittel, welches neben einer bekannten, stark herbizid wirkenden Verbindung als Gegenmittel eine Verbindung der Formel I, Anspruch 1 enthält.

3. Die Verwendung einer Verbindung der Formel

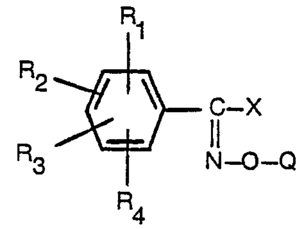

worin X, Q, $R_1$, $R_2$, $R_3$ und $R_4$ die unter Formel I, Anspruch 1 gegebene Bedeutung haben, zum Schutz von Pflanzenkulturen vor agressiven Agrarchemikalien.

4. Die Verwendung einer Verbindung der Formel

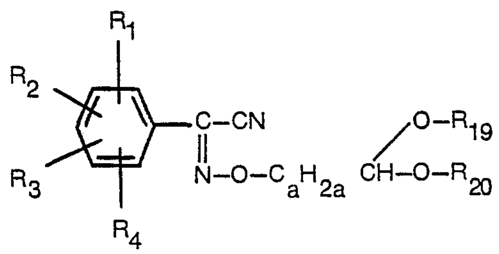

worin a, $R_1$, $R_2$, $R_3$, $R_4$, $R_{19}$ und $R_{20}$ die unter Formel I, Anspruch 1 gegebene Bedeutung haben, zur Hemmung des Pflanzenwachstums in Kulturpflanzen.

5. Die Verbindung der Formel

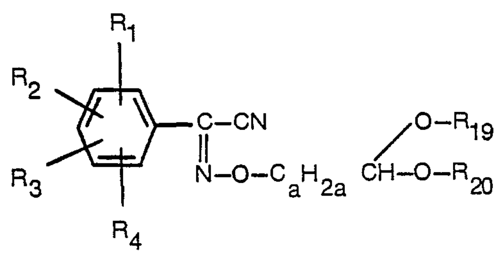

worin a, $R_1$, $R_2$, $R_3$, $R_4$, $R_{19}$ und $R_{20}$ die unter Formel I, Anspruch 1 gegebene Bedeutung haben.

6. Phenyl-acetonitriloxim-carboxylmethyl-äther.
7. Phenyl-acetonitriloxim-(2,2'-dimethoxy-äthyl)-äther.
8. Phenyl-acetonitriloxim-(N-formyl-N-methyl-carbamoylmethyl)-äther.
9. Phenyl-acetonitriloxim-(N-methoxy-carbonyl-carbamoylmethyl)-äther.
10. Phenyl-acetonitriloxim-(ureidocarbonylmethyl)-äther.
11. 4-Chlorphenyl-acetonitriloxim-(hydrazido-carbonylmethyl)-äther.
12. Phenyl-acetonitriloxim-(2'-äthylendioxy-äthyl)-äther.
13. Phenyl-acetonitriloxim-(2'-propylendioxy-äthyl)-äther.
14. Phenyl-acetonitriloxim-[2'-(2'',2''-dimethyl-propylendioxy)-äthyl]-äther.
15. Phenyl-acetonitriloxim-[2'(1'',1'',3''-trimethyl-propylendioxy)-äthyl]-äther.
16. Phenyl-acetontriloxim-(2'-methylpiperidino)-carbonylmethyl-äther.
17. 4-Tolyl-acetonitriloxim-(2',2'-dimethoxy-äthyl)-äther.
18. 3,4-Dichlorphenyl-acetonitriloxim-(2',2'-dimethyloxyäthyl)-äther.
19. 3,4-Dichlorphenyl-acetonitriloxim-[2'-(2'',2''-dimethylpropylendioxy)-äthyl]-äther.
20. 2-Chlorphenyl-acetonitriloxim-[2'-(2'',2''-dimethylpropylendioxy)-äthyl]-äther.
21. 4-Anisyl-acetonitriloxim-(3'-äthylendioxypropyl)-äther.
22. 3,4-Dichlorphenyl-acetonitriloxim-(3'-äthylendioxypropyl)-äther.
23. 2-Chlorphenyl-acetonitriloxim-(2'-äthylendioxy-äthyl-äther.
24. Phenyl-acetonitriloxim-(4'-anisyl)-carbonyl-methyl-äther.
25. 4-Anisyl-acetonitriloxim-(2'-diäthoxy-äthyl)-äther.
26. 2,4-Dichlorphenyl-acetonitriloxim-(2'-diäthoxy-äthyl)-äther.
27. Phenyl-acetonitriloxim-(3'-diäthoxy-propyl)-äther.

28. α-Naphthyl-acetonitriloxim-(3'-diäthoxy-äthyl)-äther.

29. Die Verwendung der Verbindung Phenyl-acetonitriloxim-(2'-propylendioxy-äthyl)-äther als Wirkstoff zur Wuchshemmung und Ertragsteigerung in Sojakulturen.

## Revendications

1. Procédé de protection des plantes cultivées contre l'action phytotoxique d'herbicides forts au moyen de dérivés d'oxime de formule

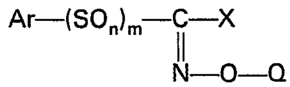

(I)

où n vaut 0, 1 ou 2 et m vaut 0 ou 1, et où

Ar représente un radical phényle

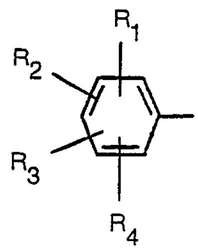

un radical naphtyle substitué par $R_2$ et $R_3$,

un radical hétérocyclique à 5 à 10 chaînons contenant au maximum 3 hétéroatomes N, O et/ou S semblables ou différents et substitué par $R_2$, $R_3$ et $R_4$ et pouvant être substitué par un oxo ou un thiono, ou bien où, lorsque m = 0,

Ar représente un radical R—CO—, où

R représente un radical —$OR_5$, où $R_5$ représente un groupe aliphatique avec au maximum 8 atomes de carbone ou un groupe araliphatique avec au maximum 15 atomes de carbone ou un groupe cycloaliphatique ou aromatique avec chacun au maximum 10 atomes de carbone, où comme substituants des radicaux aromatiques ou du radical cycloaliphatique on peut avoir un halogène, —CN, —$NO_2$, un alcoyle inférieur, un alcoxy inférieur, un halogènealcoyle,

un radical —NH—CO—NH—$R_7$ ou

un radical —$N(R_6)(R_7)$, où $R_6$ représente un hydrogène ou un alcoyle inférieur et $R_7$ représente un hydrogène ou un groupe aliphatique avec au maximum 8 atomes de carbone ou un groupe araliphatique avec au maximum 15 atomes de carbone ou un groupe cycloaliphatique ou aromatique avec chacun au maximum 10 atomes de carbone où l'on peut avoir comme substituants possibles du groupe aromatique ou du radical cycloaliphatique un halogène, —CN, $NO_2$, un alcoyle inférieur, un alcoxy inférieur, un halogènealcoyle;

un radical —$N(R_6)(R_7)$, où $R_6$ et $R_7$ forment ensemble avec l'atome d'azote un noyau hétérocyclique à 5 à 6 chaînons qui peut encore contenir un oxygène comme autre hétéroatome possible,

$R_1$ représente un hydrogène, un halogène, un alcoyle inférieur, un alcoxy inférieur ou un radical phénoxy en position para éventuellement substitué au maximum 2 fois par un halogène, CN, $NO_2$, $CF_3$,

$R_2$, $R_3$, $R_4$ représentent indépendamment l'un de l'autre un hydrogène, un halogène, CN, $NO_2$, un alcoyle inférieur, un alcoxy inférieur, un halogènealcoyle, un halogènealcoxy, un alcanoyle inférieur, OH, un phényle, un halogénophényle, un carbalcoxy inférieur, un alcoxy inférieur-carbonyle, un alcoxy inférieur-carbonyloxy, un carbamoyloxy inférieur, un alcoyle inférieur-thio, un alcoyle inférieursulfonyle, un phénalcoxy, un cyclohexyle, $NH_2$, —NH-alcoyle inférieur, —N(alcoyle inférieur)$_2$, un alcanoyle inférieur-amino, un amide d'acide carboxylique, un amide d'acide sulfonique,

X représente un hydrogène, —CN, un halogène, un alcoyle inférieur, un alcanoyle inférieur, —COOH, un radical ester d'acide carboxylique, un radical amide d'acide carboxylique, et

Q représente le radical —$C_aH_{2a}$—$R_8$, où a représente un nombre entier compris entre 1 et 6; où le radical correspondant peut également être ramifié, et $R_8$ représente l'un des groupes suivants:

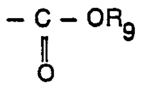 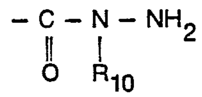 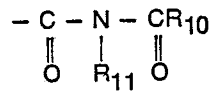 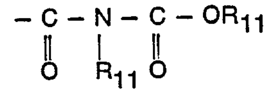

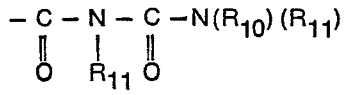 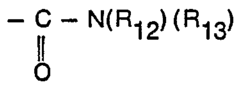 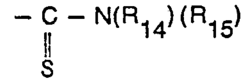

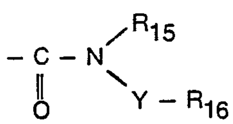 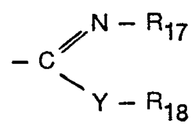 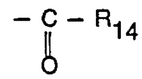 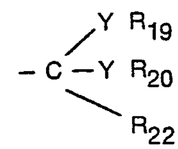

où les substituants $R_9$ à $R_{20}$, $R_{22}$ et Y ont les définitions suivantes:

$R_9$ représente un hydrogène ou le cation d'une base 1/p $M^{p\oplus}$ où

M représente un cation alcalin, alcalino-terreux ou un cation Fe, Cu, Zn, Mn, Co, Ni ou un radical amino

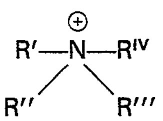

et

p en tant que nombre entier 1, 2 ou 3 tient compte de la valance du cation, tandis que R', R'', R''' et $R^{IV}$ représentent indépendamment l'un de l'autre un hydrogène, un benzyle ou un radical alcoyle en $C_1$ à $C_4$ éventuellement substitué par —OH, —$NH_2$ ou un alcoxy en $C_1$ à $C_4$,

$R_{10}$ représente un hydrogène, un radical aliphatique, un radical araliphatique ou un radical aromatique éventuellement mono- ou polysubstitué par un halogène, un halogènealcoyle, un alcoxy inférieur et/ou un cyano,

$R_{11}$ représente un hydrogène ou un alcoyle inférieur

$R_{12}$ et $R_{13}$ représentent ensemble avec l'atome d'azote un noyau à 5 à 6 chaînons éventuellement mono- ou polysubstitué par un halogène, un cyano et/ou un alcoyle inférieur et éventuellement interrompu par un atome de N, O ou S,

$R_{14}$ représente un radical cycloaliphatique ou araliphatique ou aliphatique ou un radical aromatique éventuellement mono- ou polysubstitué par un halogène, un halogènealcoyle, un alcoxy inférieur et/ou un cyano,

$R_{15}$ représente un hydrogène, un alcoyle inférieur ou un cycloalcoyle,

$R_{16}$ représente un radical aliphatique éventuellement substitué par un cyano ou un radical aromatique éventuellement mono- ou polysubstitué par un halogène, un halogènealcoyle, un alcoxy inférieur et/ou un cyano,

$R_{17}$ représente un hydrogène, un alcényle inférieur ou un alcynyle inférieur éventuellement substitué par un halogène, un alcoyle inférieur ou un cycloalcoyle,

$R_{18}$ représente un alcoyle inférieur ou un cycloalcoyle,

$R_{17}$ et $R_{18}$ forment ensemble avec —N=C—Y— un noyau à 5 à 6 chaînons éventuellement substitué par un alcoyle inférieur,

$R_{19}$ et $R_{20}$ représentent indépendamment l'un de l'autre un alcoyle inférieur ou avec —Y—C—Y un noyau à 5 à 6 chaînons éventuellement substitué par un alcoyle inférieur, un halogène et/ou un nitro.

$R_{22}$ représente un hydrogène, un radical cycloaliphatique ou araliphatique ou aliphatique ou un radical aromatique éventuellement mono- ou polysubstitué par un halogène, un halogènealcoyle, un alcoxy inférieur et/ou un cyano,

Y représente un oxygène ou un soufre.

2. Agent herbicide contenant à côté d'un composé à action fortement herbicide connue, comme antidote un composé de formule I de la revendication 1.

3. Application d'un composé de formule

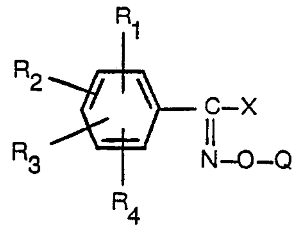

où X, Q, $R_1$, $R_2$, $R_3$ et $R_4$ ont la signification donnée pour la formule I de la revendication 1, à la protection des plantes cultivées contre les produits agrochimiques agressifs.

4. Application d'un composé de formule

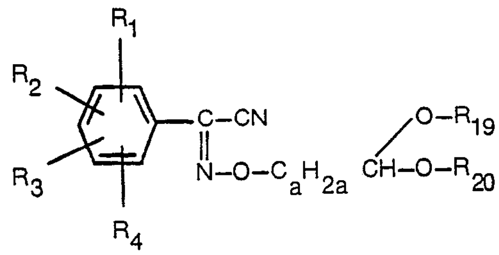

ou a, $R_1$, $R_2$, $R_3$, $R_4$, $R_{19}$ et $R_{20}$ ont la signification donnée pour la formule I de la revendication 1, à l'inhibition de la croissance végétale dans les plantes cultivées.

5. Le composé de formule

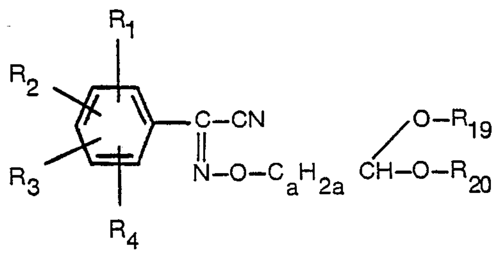

où a, $R_1$, $R_2$, $R_3$, $R_4$, $R_{19}$ et $R_{20}$ ont la signification donnée pour la formule I de la revendication 1.

6. Phényl-acétonitriloxime-carboxyméthyl-éther.
7. Phényl-acétonitriloxime-(2,2'-diméthoxy-éthyl)-éther.
8. Phényl-acétonitriloxime-(N-formyl-N-méthyl-carbamoylméthyl)-éther.
9. Phényl-acétonitriloxime-(N-méthoxy-carbonyl-carbamoylméthyl)-éther.
10. Phényle-acétonitriloxime-(uréidocarbonylméthyl)-éther.
11. 4-chlorophényl-acétonitriloxime-(hydrazido-carbonylméthyl)-éther.
12. Phényl-acétonitriloxime-(2'-éthylènedioxy-éthyl)-éther.
13. Phényl-acétonitriloxime-(2'-propylènedioxy-éthyl)-éther.
14. Phényl-acétonitriloxime-[2'-(2",2"-diméthyl-propylènedioxy)-éthyl]-éther.
15. Phényl-acétonitriloxime-[2'-(1",1",3"-triméthylpropylènedioxy)-éthyl]-éther.
16. Phényl-acétonitriloxime-(2'-méthylpipéridino)-carbonylméthyl-éther.
17. 4-tolyl-acétonitriloxime-(2',2'-diméthoxy-éthyl)-éther.
18. 3,4-dichlorophényl-acétonitriloxime-(2',2'-diméthyloxyéthyl)-éther.
19. 3,4-dichlorophényl-acétonitriloxime-[2'-(2",2"-diméthylpropylènedioxy)-éthyl]-éther.
20. 2-chlorophényl-acétonitriloxime-[2'-(2",2"-diméthylpropylènedioxy)-éthyl]-éther.
21. 4-anisyl-acétonitriloxime-(3'-éthylènedioxypropyl)-éther.
22. 3,4-dichlorophényle-acétonitriloxime-(3'-éthylènedioxypropyl)-éther.
23. 2-chlorophényl-acétonitriloxime-(2'-éthylènedioxyéthyl)-éther.
24. Phényl-acétonitriloxime-(4'-anisyl)-carbonyl-méthyléther.
25. 4-anisyl-acétonitriloxime-(2'-diéthoxy-éthyl)-éther.
26. 2,4-dichlorophényl-acétonitriloxime-(2'-diéthoxyéthyl)-éther.
27. Phényl-acétonitriloxime-(3'-diéthoxy-propyl)-éther.
28. $\alpha$-naphtyl-acétonitriloxime-(3'-diéthoxy-éthyl)-éther.

29. Application du composé phényl-acétonitriloxime-(2'-propylènedioxy-éther)-éther comme matière active pour inhiber la croissance et accroître le rendement dans les cultures de soja.

**Claims**

1. A method of protecting plant crops from the phytotoxic action of potent herbicides with an oxime derivative of the formula (I)

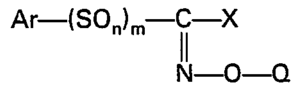

(I)

wherein

n is 0, 1 or 2 and m is 0 or 1, and

Ar is a phenyl radical

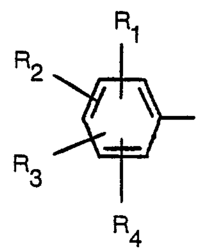

a naphthyl radical substituted by $R_2$ and $R_3$, a 5- to 10-membered hetercyclic radical which contains not more than 3 identical or different heteroatoms N, O and/or S and which is substituted by $R_2$, $R_3$ and $R_4$ and can be substituted by oxo or thiono, or if m is 0, Ar is a radical R—CO, wherein R is a radical —$OR_5$, in which $R_5$ is an aliphatic group containing not more than 8 carbon atoms or is an araliphatic group containing not more than 15 carbon atoms or is a cycloalphatic or aromatic group, each containing not more than 10 carbon atoms, the possible substituents of the aromatic radicals or of the cycloaliphatic radical being halogen, —CN, —$NO_2$, lower alkyl, lower alkoxy, haloalkyl; or R is a radical —NH—CO—NH—$R_7$ or a radical —$N(R_6)(R_7)$, wherein $R_6$ is hydrogen or lower alkyl and $R_7$ is hydrogen or an aliphatic group containing not more than 8 carbon atoms or an araliphatic group containing not more than 15 carbon atoms, or a cycloaliphatic or aromatic group containing not more than 10 carbon atoms, the possible substituents of the aromatic group or of the cycloaliphatic radical being halogen, —CN, $NO_2$, lower alkyl, lower alkoxy, or haloalkyl; or R is a radical —$N(R_6)(R_7)$, wherein $R_6$ and $R_7$ together form a 5- or 6-membered heterocyclic ring which can additionally contain oxygen as possible further heteroatom,

$R_1$ is hydrogen, halogen, lower alkyl, lower alkoxy or a p-phenoxy radical which is unsubstituted or at most disubstituted by halogen, CN, $NO_2$, $CF_3$,

$R_2$, $R_3$ and $R_4$, each independently of the other, are hydrogen, halogen, CN, $NO_2$, lower alkyl, lower alkoxy, haloalkyl, haloalkoxy, lower alkanoyl, OH, phenyl, halophenyl, lower carbalkoxy, lower alkoxycarbonyl, lower alkoxycarbonyloxy, lower carbamoyloxy, lower alkylthio, lower alkylsulfonyl, phenalkoxy, cyclohexyl, $NH_2$, —NH-lower alkyl, —N(di-lower alkyl), lower alkanoylamino, carbamoyl, sulfamoyl,

X is hydrogen, —CN, halogen, lower alkyl, lower alkanoyl, —COOH, a carboxylic acid ester radical, a carbamoyl radical, and

Q is the radical —$C_aH_{2a}$—$R_8$, wherein a is an integer from 1 to 6, and the corresponding radical can also be branched and $R_8$ is one of the following radicals:

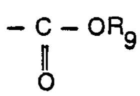 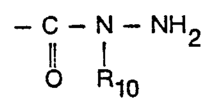 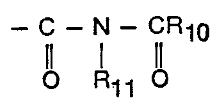 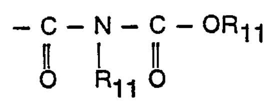

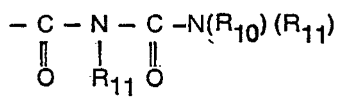 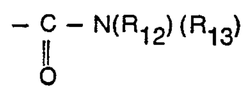 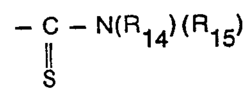

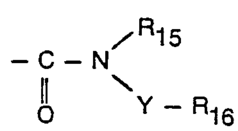 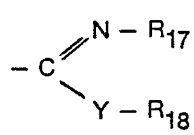 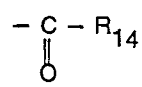 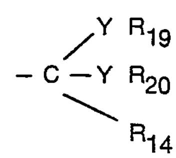

wherein $R_9$ is hydrogen or the cation of a base $\frac{1}{p} M^{p\oplus}$,

in which M is the cation of an alkali metal or alkaline earth metal or an iron, copper, zinc, manganese, cobalt or nickel cation or an amino radical

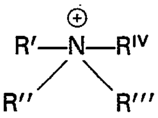

and p is an integer 1, 2 or 3 which corresponds to the valency of the cation, while R′, R″, R‴ and $R^{IV}$, each independently of the other, are hydrogen, benzyl or a $C_1$—$C_4$alkyl radical which is unsubstituted or substituted by —OH, —$NH_2$ or $C_1$—$C_4$alkoxy; $R_{10}$ is hydrogen, an aliphatic radical, an araliphatic radical or an aromatic radical which is unsubstituted or mono- or polysubstituted by halogen, haloalkyl, lower alkoxy and/or cyano; $R_{11}$ represents hydrogen or lower alkyl; $R_{12}$ and $R_{13}$ together with the nitrogen atom to which they are attached form a 5- to 6-membered ring which is unsubstituted or mono- or polysubstituted by halogen, cyano and/or lower alkyl and which can be interrupted by a nitrogen, oxygen or sulfur atom; $R_{14}$ is a cycloaliphatic, araliphatic or aliphatic radical or an aromatic radical which is unsubstituted or mono- or polysubstituted by halogen, haloalkyl, lower alkoxy and/or cyano; $R_{15}$ is hydrogen, lower alkyl or cycloalkyl; $R_{16}$ is an aliphatic radical which is unsubstituted or substituted by cyano or an aromatic radical which is unsubstituted or mono- or polysubstituted by halogen, haloalkyl, lower alkoxy and/or cyano; $R_{17}$ is hydrogen, lower alkenyl or lower alkynyl which are unsubstituted or substituted by halogen, or is lower alkyl or cycloalkyl; $R_{18}$ is lower alkyl or cycloalkyl; $R_{17}$ and $R_{18}$ together with —N=C—Y— forms a 5- to 6-membered ring which is unsubstituted or substituted by lower alkyl; $R_{19}$ and $R_{20}$, each independently of the other, are lower alkyl or together with —Y—C—Y— form a 5- to 6-membered ring which is unsubstituted or substituted by lower alkyl, halogen and/or nitro, and Y is oxygen or sulfur.

2. A herbicidal composition which, in addition to containing a known compound with a strong herbicidal action, contains a compound of the formula I according to claim 1 as antidote.

3. A method of protecting plant crops from aggressive agrochemicals, which comprises the use of a compound of the formula

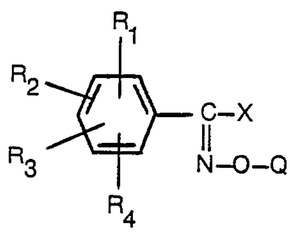

wherein X, Q, $R_1$, $R_2$, $R_3$ and $R_4$ are as defined for formula I according to claim 1.

4. A method of inhibiting plant growth in crops of cultivated plants, which comprises the use of a compound of the formula

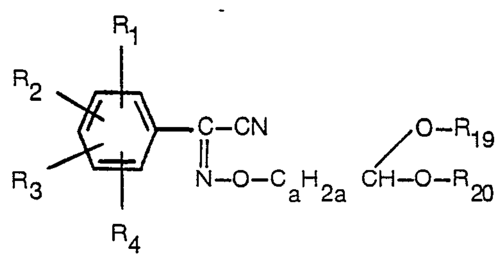

wherein a, $R_1$, $R_2$, $R_3$, $R_4$, $R_{19}$ and $R_{20}$ are as defined for formula I according to claim 1.

5. The compound of the formula

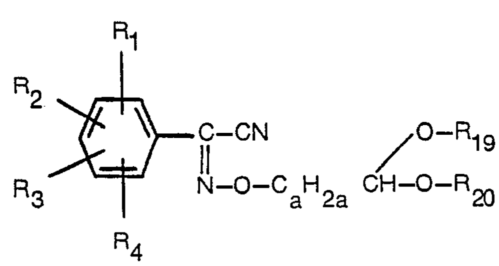

wherein a, $R_1$, $R_2$, $R_3$, $R_4$, $R_{19}$ and $R_{20}$ are as defined for formula I according to claim 1.

6. Phenylacetonitrile oxime-carboxylmethyl ether.

7. Phenylacetonitrile oxime-(2,2′-dimethoxyethyl) ether.

8. Phenylacetonitrile oxime-(N-formyl-N-methylcarbamoylmethyl) ether.
9. Phenylacetonitrile oxime-(N-methoxycarbonylcarbamoylmethyl) ether.
10. Phenylacetonitrile oxime-(ureidocarbonylmethyl) ether.
11. 4-Chlorophenylacetonitrile oxime-(hydrazidocarbonylmethyl) ether.
12. Phenylacetonitrile oxime-(2′-ethylenedioxyethyl) ether.
13. Phenylacetonitrile oxime-(2′-propylenedioxyethyl) ether.
14. Phenylacetonitrile oxime-[2′-(2″,2″-dimethylpropylenedioxy)-ethyl] ether.
15. Phenylacetonitrile oxime-[2′-(1″,1″,3″-trimethylpropylene-dioxy)-ethyl] ether.
16. Phenylacetonitrile oxime-(2′-methylpiperidino)-carbonylmethyl ether.
17. 4-Tolylacetonitrile oxime-(2′,2′-dimethoxyethyl) ether.
18. 3,4-Dichlorophenylacetonitrile oxime-(2′,2′-dimethoxyethyl) ether.
19. 3,4-Dichlorophenylacetonitrile oxime-[2′-(2″,2″-dimethylpropylenedioxy)-ethyl] ether.
20. 2-Chlorophenylacetonitrile oxime-[2′-(2″,2″-dimethylpropylene-dioxy)-ethyl] ether.
21. 4-Anisylacetonitrile oxime-(3′-ethylenedioxypropyl) ether.
22. 3,4-Dichlorophenylacetonitrile oxime-(3′-ethylenedioxypropyl) ether.
23. 2-Chlorophenylacetonitrile oxime-(2′-ethylenedioxyethyl) ether.
24. Phenylacetonitrile oxime-(4′-anisyl)-carbonylmethyl ether.
25. 4-Anisylacetonitrile oxime-(2′-diethoxyethyl) ether.
26. 2,4-Dichlorophenylacetonitrile oxime-(2′-diethoxyethyl) ether.
27. Phenylacetonitrile oxime-(3′-diethoxypropyl) ether.
28. α-Naphthylacetonitrile oxime-(3′-diethoxyethyl) ether.
29. A method of inhibiting plant growth and increasing the yield in crops of soybeans, which comprises the use of phenylacetonitrile oxime-(2′-propylenedioxyethyl) ether.